# EUROPEAN PATENT SPECIFICATION

(11) **EP 0 994 893 B1**
(45) Date of publication and mention of the grant of the patent: **02.01.2002**
(21) Application number: 98939911.8
(22) Date of filing: 11.08.1998
(51) Int. Cl.: C07K 5/06, A61K 38/04, C07K 5/02

(54) **SELECTIVE FACTOR XA INHIBITORS CONTAINING A FUSED AZEPINONE STRUCTURE**
SELEKTIVE INHIBITOREN DES FAKTORS X, EINE AZEPINONSTRUKTUR ENTHALTEND
INHIBITEURS SELECTIFS DU FACTEUR Xa CONTENANT UNE STRUCTURE D'AZEPINONE CONDENSEE

(30) Priority: 11.08.1997 US 907779; 11.08.1997 US 82316 P
(43) Date of publication of application: 26.04.2000
(73) Proprietor: COR THERAPEUTICS, INC., South San Francisco, CA 94080 (US)
(72) Inventor: SCARBOROUGH, Robert, M., Belmont, CA 94002 (US)
(74) Representative: Doireau, Marc
(86) International application number: US9816704
(87) International publication number: WO9907730

(56) References cited:
- WO-A-97/05160
- J A ROBL ET AL.: "Dual metalloprotease inhibitors. II. Effect of substitution and stereochemistry on benzazepinone based mercaptoacetyls" BIOORGANIC AND MEDICINAL CHEMISTRY LETTERS, vol. 4, no. 15, 1994, pages 1795-1800, XP000196070 Amsterdam
- J A ROBL ET AL.: "Dual metalloprotease inhibitors. 6. Incorporation of bicyclic and substituted monocyclic azepinones as dipeptide surrogates in angiotensin-converting enzyme/neutral endopeptidase inhibitors" JOURNAL OF MEDICINAL AND PHARMACEUTICAL CHEMISTRY., vol. 39, 1996, pages 494-502, XP000749701 EASTON US

## Description

### Field of the Invention

This invention relates to a novel class of fused aryl diazepinone compounds which are potent and highly selective inhibitors of factor Xa or factor Xa when assembled in the prothrombinase complex. These compounds show selectivity for factor Xa versus other proteases of the coagulation (e.g. thrombin, fVIIa. fIXa) or the fibrinolytic cascades (e.g. plasminogen activators, plasmin).

### Background of the Invention

Blood coagulation protects mammalian species when the integrity of the blood vessel wall is damaged and uncontrolled loss of blood threatens survival. Coagulation, resulting in the clotting of blood, is an important component of hemostasis. Under normal hemostatic circumstances, there is maintained an acute balance of clot formation and clot removal (fibrinolysis). The blood coagulation cascade involves the conversion of a variety of inactive enzymes (zymogens) into active enzymes, which ultimately convert the soluble plasma protein fibrinogen into an insoluble matrix of highly cross-linked fibrin. (See Davie, *et al*., "The Coagulation Cascade: Initiation, Maintenance and Regulation" Biochemistry 30:10363-10370 (1991)). Blood platelets which adhere to damaged blood vessels are activated and incorporated into the clot and thus play a major role in the initial formation and stabilization of hemostatic "plugs". In certain diseases of the cardiovascular system, deviations from normal hemostasis push the balance of clot formation and clot dissolution towards life-threatening thrombus formation when thrombi occlude blood flow in coronary vessels (myocardial infarctions) or limb and pulmonary veins (venous thrombosis). Although platelets and blood coagulation are both involved in thrombus formation, certain components of the coagulation cascade are primarily responsible for the amplification or acceleration of the processes involved in platelet aggregation and fibrin deposition.

A key enzyme in the coagulation cascade, as well as in hemostasis, is thrombin. Thrombin is intimately involved in the process of thrombus formation, but under normal circumstances can also play an anticoagulant role in hemostasis through its ability to convert protein C into activated protein C in a thrombomodulin-dependent manner. Thrombin plays a central role in thrombosis through its ability to catalyze the penultimate conversion of fibrinogen into fibrin and through its potent platelet activation activity. Direct or indirect inhibition of thrombin activity has been the focus of a variety of recent anticoagulant strategies as reviewed by Claeson "Synthetic Peptides and Peptidomimetics as Substrates and Inhibitors of Thrombin and Other Proteases in the Blood Coagulation System", Blood Coag. Fibrinol. 5:411-436 (1994). More recently, it was proposed in WO 97/05160 to use, as thrombin inhibitors, compounds formed by a bicyclic lactam residue which is capable of acting as a conformationally constricted analogue of a peptidic sequence such as the one consisting of Phe-Pro-Arg, which is present in fibrinogen structure and is considered important for recognizing the thrombin active site, the bicyclic lactam residue being further susbstituted by an arginine residue. The major classes of anticoagulants currently used in the clinic directly or indirectly affect thrombin (i.e. heparins, lowmolecular weight heparins and coumarins). Thrombin is generated at the convergence of the intrinsic and extrinsic coagulation pathways by the prothrombinase complex. The prothrombinase complex is formed when activated Factor X (factor Xa) and its nonenzymatic cofactor, factor Va assemble on phospholipid surfaces in a Ca+²-dependent fashion as reviewed by Mann, *et al*., "Surface-Dependent Reactions of the Vitamin K-Dependent Enzymes", Blood 76:1-16 (1990). The prothrombinase complex converts the zymogen prothrombin into the active procoagulant thrombin. The location of the prothrombinase complex at the convergence of the intrinsic and extrinsic coagulation pathways, and the significant amplification of thrombin generation (393,000-fold over uncomplexed factor Xa) mediated by the complex at a limited number of targeted catalytic units present at vascular lesion sites, suggests that inhibition of thrombin generation is an ideal method to block uncontrolled procoagulant activity. Unlike thrombin, which acts on a variety of protein substrates as well as at a specific receptor, factor Xa appears to have a single physiologic substrate, namely prothrombin. Plasma contains an endogenous inhibitor of both the factor VIIa-tissue factor (TF) complex and factor Xa called tissue factor pathway inhibitor (TFPI). TFPI is a Kunitz-type protease inhibitor with three tandem Kunitz domains. TFPI inhibits the TF/fVIIa complex in a two-step mechanism which includes the initial interaction of the second Kunitz domain of TFPI with the active site of factor Xa, thereby inhibiting the proteolytic activity of factor Xa. The second step involves the inhibition of the TF/fVIIa complex by formation of a quaternary complex TF/fVIIa/TFPI/fXa as described by Girard, *et al*., "Functional Significance of the Kunitz-type Inhibitory Domains of Lipoprotein-associated Coagulation Inhibitor", Nature 338:518-520 (1989).

Polypeptides derived from hematophagous organisms have been reported which are highly potent and specific inhibitors of factor Xa. U.S. Pat. No. 4,588,587 awarded to Gasic, describes anticoagulant activity in the saliva of the Mexican leech, *Haementeria officinalis*. A principal component of this saliva is shown to be the polypeptide factor Xa inhibitor, antistasin, by Nutt, *et al*., "The Amino Acid Sequence of Antistasin, a Potent Inhibitor of Factor Xa Reveals a Repeated Internal Structure", J. Biol. Chem. 263:10162-10167 (1988).

Another potent and highly specific inhibitor of Factor Xa, tick anticoagulant peptide, has been isolated from the whole body extract of the soft tick *Ornithidoros moubata*, as reported by Waxman, *et al*., "Tick Anticoagulant Peptide (TAP) is a Novel Inhibitor of Blood Coagulation Factor Xa", Science 248:593-596 (1990).

Other polypeptide type inhibitors of factor Xa have been reported including the following citations by: Condra, *et al*., "Isolation and Structural Characterization of a Potent Inhibitor of Coagulation Factor Xa from the Leech *Haementeria ghilianii*", Thromb. Haemost. 61:437-441 (1989); Blankenship, *et al*., "Amino Acid Sequence of Ghilanten: Anti-coagulant-antimetastatic Principle of the South American Leech, *Haementeria ghilianii*", Biochem. Biophys. Res. Commun. 166:1384-1389 (1990); Brankamp, *et al*., "Ghilantens: Anticoagulants, Antimetastatic Proteins from the South American Leech *Haementeria ghilianii*", J. Lab. Clin. Med. 115:89-97 (1990); Jacobs, *et al*., "Isolation and Characterization of a Coagulation Factor Xa Inhibitor from Black Fly Salivary Glands", Thromb. Haemost. 64:235-238 (1990); Rigbi, *et al*., "Bovine Factor Xa Inhibiting Factor and Pharmaceutical Compositions Containing the Same", European Patent Application, 352,903 (1990); Cox, "Coagulation Factor X Inhibitor From the Hundred-pace Snake *Deinagkistrodon acutus* venom", Toxicon 31:1445-1457 (1993); Cappello, *et al*., "*Ancylostoma* Factor Xa Inhibitor: Partial Purification and its Identification as a Major Hookworm-derived Anticoagulant *In Vitro",* J. Infect. Dis. 167:1474-1477 (1993); Seymour, *et al*., "Ecotin is a Potent Anticoagulant and Reversible Tight-binding Inhibitor of Factor Xa", Biochemistry 33:3949-3958 (1994).

Factor Xa inhibitory compounds which are not large polypeptide-type inhibitors have also been reported including: Tidwell, *et al*., "Strategies for Anticoagulation With Synthetic Protease Inhibitors. Xa Inhibitors Versus Thrombin Inhibitors", Thromb. Res. 19:339-349 (1980); Turner, *et al*., "p-Amidino Esters as Irreversible Inhibitors of Factor IXa and Xa and Thrombin", Biochemistry 25:4929-4935 (1986); Hitomi, *et al*., "Inhibitory Effect of New Synthetic Protease Inhibitor (FUT-175) on the Coagulation System", Haemostasis 15:164-168 (1985); Sturzebecher, *et al*., "Synthetic Inhibitors of Bovine Factor Xa and Thrombin. Comparison of Their Anticoagulant Efficiency", Thromb. Res. 54:245-252 (1989); Kam, *et al*., "Mechanism Based Isocoumarin Inhibitors for Trypsin and Blood Coagulation Serine Proteases: New Anticoagulants", Biochemistry 27:2547-2557 (1988); Hauptmann, *et al*., "Comparison of the Anticoagulant and Antithrombotic Effects of Synthetic Thrombin and Factor Xa Inhibitors", Thromb. Haemost. 63:220-223 (1990); Miyadera, *et al*., Japanese Patent Application JP 6327488 (1994); Nagahara, *et al*., "Dibasic (Amidinoaryl)propanoic Acid Derivatives as Novel Blood Coagulation Factor Xa Inhibitors", J. Med. Chem. 37:1200-1207 (1994); Vlasuk, *et al*., "Inhibitors of Thrombosis", WO 93/15756; and Brunck, *et al*., "Novel Inhibitors of Factor Xa", WO 94/13693. Al-obeidi, *et al*., "Factor Xa Inhibitors", WO 95/29189, discloses pentapeptide X1-Y-I-R-X2 derivatives as factor Xa inhibitors. Said compounds are useful for inhibiting blood clotting in the treatment of thrombosis, stroke, and myocardial infarction.

### Summary Of The Invention

The present invention relates to novel peptide mimetic analogs, their pharmaceutically acceptable isomers, salts, hydrates, solvates and prodrug derivatives.

In another aspect, the present invention includes pharmaceutical compositions comprising a pharmaceutically effective amount of the compounds of this invention and a pharmaceutically acceptable carrier. These compositions are useful as potent and specific inhibitors of blood coagulation in mammals.

In yet another aspect, the invention relates to methods of using these inhibitors as therapeutic agents for disease states in mammals which have disorders of coagulation such as in the treatment or prevention of unstable angina, refractory angina, myocardial infarction, transient ischemic attacks, thrombotic stroke, embolic stroke, disseminated intravascular coagulation including the treatment of septic shock, deep venous thrombosis in the prevention of pulmonary embolism or the treatment of reocclusion or restenosis of reperfused coronary arteries. These compositions may optionally include anticoagulants, antiplatelet agents, and thrombolytic agents.

In other aspects of the invention compounds are provided which are useful as diagnostic reagents.

In preferred embodiments, the present invention provides compounds of general formula I: 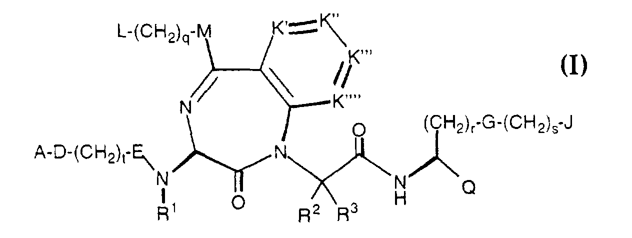 Wherein:
R¹ and R² are independently selected from the group consisting of H, C₁₋₆alkyl, C₃₋₈cycloalkyl, C₁₋₃alkylaryl, C₁₋₃alkyl-C₃₋₈cycloalkyl and aryl;
R³ is H, C₁₋₆alkyl, or R² and R³ are taken together to form a carbocyclic ring:
q is an integer from 0-1;
r is an integer from 0-4;
s is an integer from 0-1;
t is an integer from 0-4;
A is selected from the group consisting of R⁸, -NR⁸R⁹, 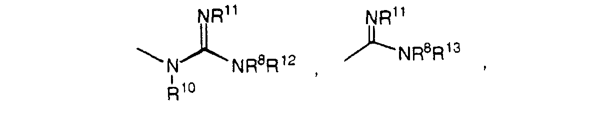 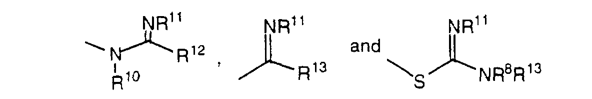 where R⁸, R⁹, R¹⁰ and R¹ are independently selected from the group consisting of H, -OH, C₁₋₆alkyl, aryl and C₁₋₄alkylaryl; R¹² is selected from the group consisting of H, C₁₋₆alkyl, aryl and C₁₋₄alkylaryl, or can be taken together with R¹⁰ or R¹¹ to form a 5-6 membered ring; and R¹³ is selected from the group consisting of H, C₁₋₆alkyl, aryl and C₁₋₄alkylaryl, or can be taken together with R¹¹ to form a 5-6 membered ring;
D is selected from the group consisting of a direct link, C₃₋₈cycloalkyl, C₁₋₆alkenyl, C₁₋ ₆alkenylaryl, aryl and a five to ten membered heterocyclic ring system containing 1-4 heteroatoms selected from the group consisting of N, O and S;
E is selected from the group consisting of a direct link, -CO-, -SO₂-, -O-CO-, -NR¹⁴-SO₂- and -NR¹⁴-CO-, where R¹⁴ is selected from the group consisting of H, -OH, C₁₋₆alkyl, aryl and C₁₋₄alkylaryl;
G is selected from the group consisting of a direct link, C₃₋₈cycloalkyl, aryl, and a five to ten membered heterocyclic ring system containing 1-4 heteroatoms selected from the group consisting of N, O and S;
J is selected from the group consisting of R¹⁵, -NR¹⁵R¹⁶, 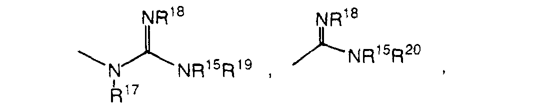 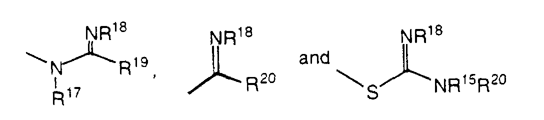 where R¹⁵, R¹⁶, R¹⁷ and R¹⁸ are independently selected from the group consisting of H, -OH, C₁₋₆alkyl, aryl and C₁₋₄alkylaryl; R¹⁹ is selected from the group consisting of H, C₁₋₆alkyl, aryl and C₁₋₄alkylaryl, or can be taken together with R¹⁷ or R¹⁸ to form a 5-6 membered ring; and R²⁰ is selected from the group consisting of H, C₁₋₆alkyl, aryl and C₁₋₄alkylaryl, or can be taken together with R¹⁸ to form a 5-6 membered ring; with the proviso that when J is R¹⁵, then G must contain at least one N atom;
K', K", K"' and K"" are independently selected from the group consisting of -CH-, -CR⁴-, -CR⁵- and -N-; with the proviso that no more than one of K', K", K"' and K"" are -CR⁴- and no more than one of K', K", K"' and K"" are -CR⁵-;
R⁴ and R⁵ are independently selected from the group consisting of C₁₋₆alkyl, aryl, C₁₋₆alkylaryl, C₁₋₄alkyloxy, halogen, -NO₂, -NR⁶R⁷, -NR⁶COR⁷, -OR⁶, -OCOR⁶, -COOR⁶, -CONR⁶R⁷, -CN, -CF₃, -SO₂NR⁶R⁷ and C₁₋₆alkyl-OR⁶; where R⁶ and R⁷ are independently selected from the group consisting of H, C₁₋₆alkyl, C₁₋₃alkylaryl and aryl;
L is selected from the group consisting of R³³, -NR³³R³⁴, 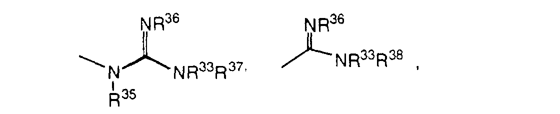 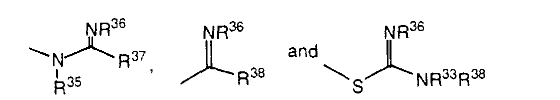 where R³³, R³⁴, R³⁵ and R³⁶ are independently selected from the group consisting of H, -OH, C₁₋₆alkyl, aryl and C₁₋₄alkylaryl; R³⁷ is selected from the group consisting of H, C₁₋₆alkyl, aryl and C₁₋₄alkylaryl, or can be taken together with R³⁵ or R³⁶ to form a 5-6 membered ring; and R³⁸ is selected from the group consisting of H, C₁₋₆alkyl, aryl and C₁₋₄alkylaryl, or can be taken together with R³⁶ to form a 5-6 membered ring;
M is selected from the group consisting of a direct link, C₁₋₆alkyl, C₃₋₈cycloalkyl, C₁₋₆alkenyl, C₁₋₆alkenylaryl, aryl, and a five to ten membered heterocyclic ring system containing 1-4 heteroatoms selected from the group consisting of N, O and S;
Q is selected from the group consisting of H, 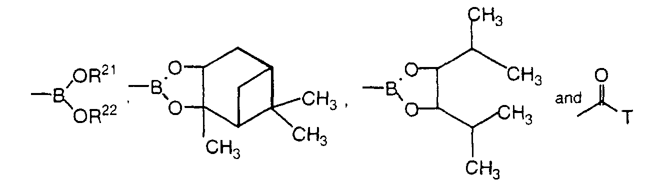 where R²¹ and R²² are independently selected from the group consisting of H, C₁₋₃alkyl and aryl; and T is selected from the group consisting of H, -COOR²³, -CONR²³R²⁴, -CF₃, -CF₂CF₃ and a group having the formula: 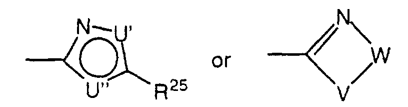 where: R²³ and R²⁴ are independently selected from the group consisting of H, C₁₋₆alkyl, aryl and C₁₋₄alkylaryl; U' and U" are independently selected from the group consisting of-O-, -S-, -N- and -NH-; with the proviso that at least one of U' or U" is -N- or -NH-; R²⁵ is selected from the group consisting of H, C₁₋₆alkyl, C₂₋₆alkenyl, C₀₋₆alkylaryl, C₂₋₆alkenylaryl, C₀₋₆alkylheterocyclo, C₂₋₆alkenylheterocyclo, -CF₃ and -CF₂CF₃; V is selected from the group consisting of -S-, -SO-, -SO₂-, -O- and -NR²⁶-, where R²⁶ is selected from the group consisting of H, C₁₋₆alkyl and benzyl; and W is selected from the group consisting of: 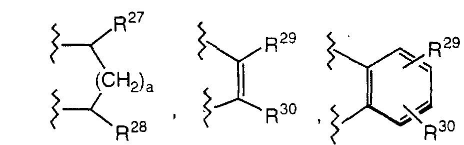 and a C₆₋₁₀ heterocyclic ring system substituted by R²⁹ and R³⁰ and containing 1-4 heteroatoms selected from N, S and O; where a: is an integer from 0-2; R²⁷ and R²⁸ are independently selected from the group consisting of H, C₁₋₆alkyl, aryl, C₁₋₆alkylaryl, -COOR³¹, -CONR³¹R³², -CN and -CF₃; and R²⁹ and R³⁰ are independently selected from the group consisting of H, C₁₋₆alkyl, aryl, C₁₋₆alkylaryl, C₁₋₄alkyloxy, halogen, -NO₂, -NR³¹R³², -NR³¹COR³², -OR³¹, -OCOR³¹, -COOR³¹, -CONR³¹R³², -CN, -CF₃, -SO₂NR³¹R³² and C₁₋₆alkyl-OR³¹; where R³¹ and R³² are independently selected from the group consisting of H, C₁₋₆alkyl, C₁₋₃alkylaryl and aryl;
and all pharmaceutically acceptable salts and optical isomers thereof.

### Detailed Description Of The Invention

### Definitions

In accordance with the present invention and as used herein, the following terms are defined with the following meanings, unless explicitly stated otherwise.

The term "alkyl" refers to saturated aliphatic groups including straight-chain, branched-chain, cyclic groups, and combinations thereof, having the number of carbon atoms specified, or if no number is specified, having up to 12 carbon atoms. The term "cycloalkyl" refers to a mono-, bi-, or tricyclic aliphatic ring having 3 to 12 carbon atoms, preferably 3 to 7 carbon atoms.

The term "alkenyl" refers to unsaturated aliphatic groups including straight-chain, branched-chain, cyclic groups, and combinations thereof, having at least one double bond and having the number of carbon atoms specified.

The term "aryl" refers to an unsubstituted or substituted aromatic ring(s), substituted with one, two or three substituents such as, by way of example and not limitation, C₁₋₆alkoxy, C₁₋₆ alkyl, C₁₋₆ alkylamino, hydroxy, halogen, cyano (-CN), mercapto, nitro (-NO₂), thioalkoxy, carboxaldehyde, carboxyl, carboalkoxy, carboxamide, -NR'R", -NR'COR", -OR, -OCOR, -COOR, -CONR'R", -CF₃, -SO₂NR'R" and C₁₋₆alkyl-OR; aryl, C₁₋₆alkylaryl (where the R groups can be H, C₁₋₆alkyl, C₁₋₃alkylaryl and aryl), including but not limited to carbocyclic aryl, heterocyclic aryl, biaryl and triaryl groups and the like, all of which may be optionally substituted. Preferred aryl groups include phenyl, halophenyl, C₁₋₆alkylphenyl, naphthyl, biphenyl, phenanthrenyl, naphthacenyl, and aromatic heterocyclics or heteroaryls, the latter of which is an aryl group containing one to four heteroatoms selected from the group consisting of nitrogen, oxygen and sulfur. Aryl groups preferably have 5-14 carbon atoms making up the ring(s) structure, while heteroaryls preferably have 1-4 heteroatoms, with the remaining 4-10 atoms being carbon atoms.

The terms "heterocyclo" and "heterocyclic ring system" as used herein refer to any saturated or unsaturated mono- or bicyclic ring system, containing from one to four heteroatoms, selected from the group consisting of nitrogen, oxygen and sulfur. A typical heterocyclic ring system will have five to ten members, 1-4 of which are heteroatoms. Typical examples of monocyclic ring systems include piperidinyl, pyrrolidinyl, pyridinyl, piperidonyl, pyrrolidonyl and thiazolyl, while examples of bicyclic ring systems include benzimidazolyl, benzothiazolyl and benzoxazolyl, all of which may be substituted.

The term "carbocyclic ring" as used herein refers to any saturated or unsaturated ring containing from three to six carbon atoms.

The terms "alkylaryl" and "alkenylaryl" as used herein refer to an alkyl group or alkenyl group, respectively, having the number of carbon atoms designated, appended to one, two, or three aryl groups. The term benzyl as used herein refers to -CH₂-C₆H₅.

The term "alkyloxy" as used herein refers to an alkyl group linked to an oxygen atom, such as methoxy, ethoxy, and so forth.

The term "halogen" as used herein refer to Cl, Br, F or I substituents.

The term "direct link" as used herein refers to a bond directly linking the substituents on each side of the direct link. When two adjacent substituents are defined as each being a "direct link", it is considered to be a single bond.

Two substituents are "taken together to form a 5-6 membered ring" means that an ethylene or a propylene bridge, respectively, is formed between the two substituents.

The term "pharmaceutically acceptable salts" includes salts of compounds derived from the combination of a compound and an organic or inorganic acid. These compounds are useful in both free base and salt form. In practice, the use of the salt form amounts to use of the base form; both acid and base addition salts are within the scope of the present invention.

"Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

"Pharmaceutically acceptable base addition salts" include those derived from inorganic bases such as sodium, potassium, lithium, ammonium, calcium, magnesium, iron, zinc, copper, manganese, and aluminum bases, and the like. Particularly preferred are the ammonium, potassium, sodium, calcium and magnesium salts. Salts derived from pharmaceutically acceptable organic nontoxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, polyamine resins and the like. Particularly preferred organic nontoxic bases are isopropylamine, diethylamine, ethanolamine, trimethamine, dicyclohexylamine, choline, and caffeine.

"Biological property" for the purposes herein means an *in vivo* effector or antigenic function or activity that is directly or indirectly performed by a compound of this invention. Effector functions include receptor or ligand binding, any enzyme activity or enzyme modulatory activity, any carrier binding activity, any hormonal activity, any activity in promoting or inhibiting adhesion of cells to an extracellular matrix or cell surface molecules, or any structural role. Antigenic functions include possession of an epitope or antigenic site that is capable of reacting with antibodies raised against it. The biological properties of the compounds of the present invention can be readily characterized by the methods described in Examples 6 and 7 and by such other methods as are well known in the art.

In addition, the following abbreviations are used in this application:
"Bn" refers to benzyl.
"Boc" refers to t-butoxycarbonyl.
"BOP" refers to benzotriazol-1-yloxy-tris-(dimethylamino) phosphonium hexafluorophosphate.
"Bu" refers to butyl.
"DCM" refers to dichloromethane.
"DIEA" refers to diisopropylethylamine.
"DMF" refers to N,N-dimethylformamide.
"Et" refers to ethyl.
"Et₂O" refers to diethyl ether.
"EtOAc" refers to ethyl acetate.
"Fmoc" refers to 9-fluorenylmethyloxycarbonyl.
"HBTU" refers to O-(1H-benzotriazol-1-yl)-1,1,3,3-tetramethyl-uronium hexafluorophosphate.
"HF" refers to hydrogen fluoride.
"Me" refers to methyl.
"MeOH" refers to methanol.
"Ph" refers to phenyl.
"TFA" refers to trifluoroacetic acid.
"THF" refers to tetrahydrofuran.
"Tos" refers to p-toluenesulfonyl.

In the compounds of this invention, carbon atoms bonded to four non-identical substituents are asymmetric. Accordingly, the compounds may exist as diastereoisomers, enantiomers or mixtures thereof. The syntheses described herein may employ racemates, enantiomers or diastereomers as starting materials or intermediates. Diastereomeric products resulting from such syntheses may be separated by chromatographic or crystallization methods, or by other methods known in the art. Likewise, enantiomeric product mixtures may be separated using the same techniques or by other methods known in the art. Each of the asymmetric carbon atoms, when present in the compounds of this invention, may be in one of two configurations (R or S) and both are within the scope of the present invention. In the processes described above, the final products may, in some cases, contain a small amount of diastereomeric or enantiomeric products; however, these products do not affect their therapeutic or diagnostic application.

In all of the peptides of the invention, one or more amide linkages (-CO-NH-) may optionally be replaced with another linkage which is an isostere such as -CH₂NH-, -CH₂S-, -CH₂-O-, -CH₂CH₂-, -CH=CH- (cis and trans), -COCH₂-, -CH(OH)CH₂-, -CH₂SO-, and -CH₂SO₂-. This replacement can be made by methods known in the art. The following references describe preparation of peptide analogs which include these alternative-linking moieties: Spatola, "Peptide Backbone Modifications" (general review) Vega Data, Vol. 1, Issue 3, (March 1983); Spatola, "Chemistry and Biochemistry of Amino Acids, Peptides and Proteins," (general review) B. Weinstein, eds., Marcel Dekker, New York, p. 267 (1983); Morley, Trends Pharm. Sci. (general review) pp. 463-468 (1980); Hudson, *et al.,* Int. J. Pept. Prot. Res. 14:177-185 (1979) (-CH₂NH-, -CH₂CH₂-); Spatola, *et al*., Life Sci. 38:1243-1249 (1986) (-CH₂-S); Hann, J. Chem. Soc. Perkin Trans. I pp.307-314 (1982) (-CH=CH-, cis and trans); Almquist, *et al*., J. Med. Chem. 23:1392-1398 (1980) (-COCH₂-); Jennings-White, *et al*., Tetrahedron Lett. 23:2533 (-COCH₂-) (1982); Szelke, *et al*., European Application EP 45665; CA:97:39405 (1982) (-CH(OH)CH₂-); Holladay, *et al.*, Tetrahedron Lett 24:4401-4404 (1983) (-CH(OH)CH₂-); and Hruby, Life Sci. 31:189-199 (1982) (-CH₂-S-).

### Preferred Embodiments

This invention relates to a new class of fused aryl diazepinone compounds selected from those of general formula I which are potent and specific inhibitors of Xa, their pharmaceutically acceptable compositions thereof, and the methods of using them as therapeutic agents for disease states in mammals characterized by abnormal thrombosis: 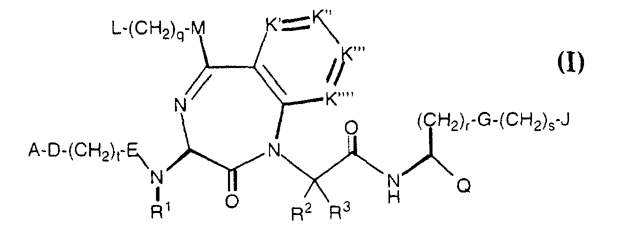 Wherein:
R¹ and R² are independently selected from the group consisting of H, C₁₋₆alkyl, C₃₋₈cycloalkyl, C₁₋₃alkylaryl, C₁₋₃alkyl-C₃₋₈cycloalkyl and aryl;
R³ is H, C₁₋₆alkyl, or R² and R³ are taken together to form a carbocyclic ring;
q is an integer from 0-1;
r is an integer from 0-4;
s is an integer from 0-1;
t is an integer from 0-4:
A is selected from the group consisting of R⁸, -NR⁸R⁹, 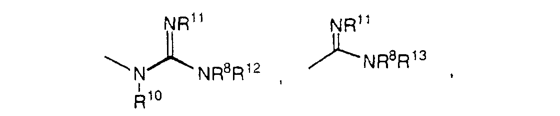 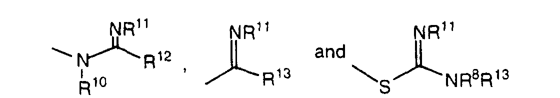 where R⁸, R⁹, R¹⁰ and R¹¹ are independently selected from the group consisting of H, -OH, C₁₋₆alkyl, aryl and C₁₋₄alkylaryl; R¹² is selected from the group consisting of H, C₁₋₆alkyl, aryl and C₁₋₄alkylaryl, or can be taken together with R¹⁰ or R¹¹ to form a 5-6 membered ring; and R¹³ is selected from the group consisting of H, C₁₋₆alkyl, aryl and C₁₋₄alkylaryl, or can be taken together with R¹¹ to form a 5-6 membered ring;
D is selected from the group consisting of a direct link, C₃₋₈cycloalkyl, C₁₋₆alkenyl, C₁₋ ₆alkenylaryl, aryl and a five to ten membered heterocyclic ring system containing 1-4 heteroatoms selected from the group consisting of N, O and S;
E is selected from the group consisting of a direct link, -CO-, -SO₂-, -O-CO-, -NR¹⁴-SO₂- and -NR¹⁴-CO-, where R¹⁴ is selected from the group consisting of H, -OH, C₁₋₆alkyl, aryl and C₁₋₄alkylaryl;
G is selected from the group consisting of a direct link, C₃₋₈cycloalkyl, aryl, and a five to ten membered heterocyclic ring system containing 1-4 heteroatoms selected from the group consisting of N, O and S;
J is selected from the group consisting of R¹⁵, -NR¹⁵R¹⁶, 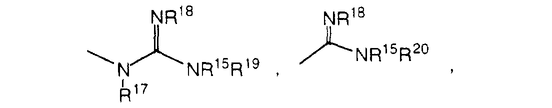 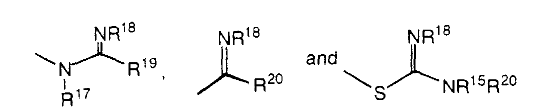 where R¹⁵, R¹⁶, R¹⁷ and R¹⁸ are independently selected from the group consisting of H, -OH, C₁₋₆alkyl, aryl and C₁₋₄alkylaryl; R¹⁹ is selected from the group consisting of H, C₁₋₆alkyl, aryl and C₁₋₄alkylaryl, or can be taken together with R¹⁷ or R¹⁸ to form a 5-6 membered ring; and R²⁰ is selected from the group consisting of H, C₁₋₆alkyl, aryl and C₁₋₄alkylaryl, or can be taken together with R¹⁸ to form a 5-6 membered ring; with the proviso that when J is R¹⁵, then G must contain at least one N atom;
K', K", K"' and K"" are independently selected from the group consisting of -CH-, -CR⁴-, -CR⁵- and -N-; with the proviso that no more than one of K', K", K"' and K"" are -CR⁴- and no more than one of K', K", K"' and K"" are -CR⁵-;
R⁴ and R⁵ are independently selected from the group consisting of C₁₋₆alkyl, aryl, C₁₋₆alkylaryl, C₁₋₄alkyloxy, halogen, -NO₂, -NR⁶R⁷, -NR⁶COR⁷, -OR⁶, -OCOR⁶, -COOR⁶, -CONR⁶R⁷, -CN, -CF₃, -SO₂NR⁶R⁷ and C₁₋₆alkyl-OR⁶; where R⁶ and R⁷ are independently selected from the group consisting of H, C₁₋₆alkyl, C₁₋₃alkylaryl and aryl;
L is selected from the group consisting of R³³, -NR³³R³⁴, 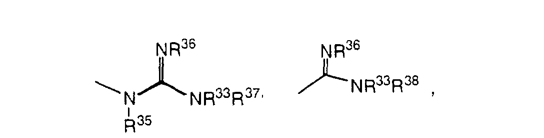 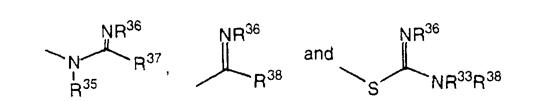 where R³³, R³⁴, R³⁵ and R³⁶ are independently selected from the group consisting of H, -OH, C₁₋₆alkyl, aryl and C₁₋₄alkylaryl; R³⁷ is selected from the group consisting of H, C₁₋₆alkyl, aryl and C₁₋₄alkylaryl, or can be taken together with R³⁵ or R³⁶ to form a 5-6 membered ring; and R³⁸ is selected from the group consisting of H, C₁₋₆alkyl, aryl and C₁₋₄alkylaryl, or can be taken together with R³⁶ to form a 5-6 membered ring;
M is selected from the group consisting of a direct link, C₁₋₆alkyl, C₃₋₈cycloalkyl, C₁₋₆alkenyl, C₁₋₆alkenylaryl, aryl, and a five to ten membered heterocyclic ring system containing 1-4 heteroatoms selected from the group consisting of N, O and S;
Q is selected from the group consisting of H, 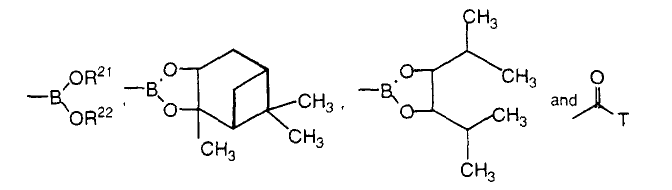 where R²¹ and R²² are independently selected from the group consisting of H, C₁₋₃alkyl and aryl; and T is selected from the group consisting of H, -COOR²³, -CONR²³R²⁴, -CF₃, -CF₂CF₃ and a group having the formula: 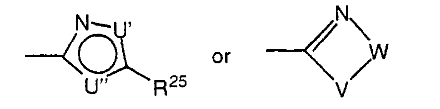 where: R²³ and R²⁴ are independently selected from the group consisting of H, C₁₋₆alkyl, aryl and C₁₋₄alkylaryl; U' and U" are independently selected from the group consisting of-O-, -S-, -N- and -NH-; with the proviso that at least one of U' or U" is -N- or -NH-; R²⁵ is selected from the group consisting of H, C₁₋₆alkyl, C₂₋₆alkenyl, C₀₋₆alkylaryl, C₂₋₆alkenylaryl, C₀₋₆alkylheterocyclo, C₂₋₆alkenylheterocyclo, -CF₃ and -CF₂CF₃; V is selected from the group consisting of -S-, -SO-, -SO₂-, -O- and -NR²⁶-, where R²⁶ is selected from the group consisting of H, C₁₋₆alkyl and benzyl; and W is selected from the group consisting of: 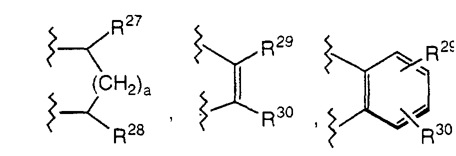 and a C₆₋₁₀ heterocyclic ring system substituted by R²⁹ and R³⁰ and containing 1-4 heteroatoms selected from N, S and O; where a: is an integer from 0-2: R²⁷ and R²⁸ are independently selected from the group consisting of H, C₁₋₆alkyl, aryl, C₁₋₆alkylaryl, -COOR³¹, -CONR³¹R³², -CN and -CF₃; and R²⁹ and R³⁰ are independently selected from the group consisting of H, C₁₋₆alkyl, aryl, C₁₋₆alkylaryl, C₁₋₄alkyloxy, halogen, -NO₂, -NR³¹R³², -NR³¹COR³², -OR³¹, -OCOR³¹, -COOR³¹, -CONR³¹R³², -CN, -CF₃, -SO₂NR³¹R³² and C₁₋₆alkyl-OR³¹; where R³¹ and R³² are independently selected from the group consisting of H, C₁₋₆alkyl, C₁₋₃alkylaryl and aryl;
and all pharmaceutically acceptable salts and optical isomers thereof.

A preferred embodiment of compounds of general structural formula I have the following stereochemistry:

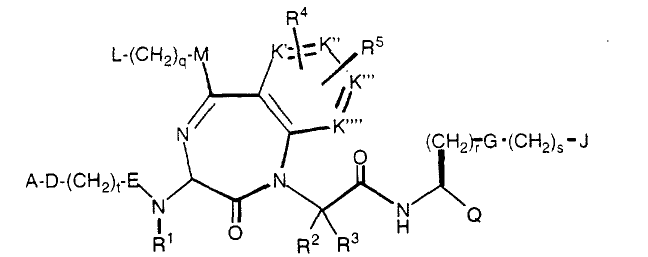

Preferred R¹ and R² substituents are H and C₁₋₆alkyl; more preferably H and methyl; most preferably H. R³ is preferably H.

The integer "r" is preferably 3.

The integer "s" is preferably 0.

The integer "t" is preferably from 0-1.

In the various "A" substituents, it is preferred that R⁸, R⁹, R¹⁰ and R¹¹ are independently selected from the group consisting of H and C₁₋₆alkyl; and are more preferably independently selected from the group consisting of H and methyl. It is also preferred that R¹² is H, C₁₋₆alkyl or taken together with R¹⁰ or R¹¹ to form a 5-6 membered ring; and is more preferably H or methyl. It is also preferred that R¹³ is H, C₁₋₆alkyl or taken together with R¹⁰ to form a 5-6 membered ring; and is more preferably H or methyl.

D is preferably selected from the group consisting of a direct link, C₃₋₈cycloalkyl, aryl and a five to ten membered heterocyclic ring system containing 1-4 heteroatoms selected from the group consisting of N, O and S.

E is preferably a direct link, -CO- or -SO₂-.

G is preferably a direct link.

In the "J" substituent, it is preferred that R¹⁵, R¹⁶, R¹⁷, R¹⁸, R¹⁹ and R²⁰ are independently selected from the group consisting of H and C₁₋₆alkyl, more preferably H and methyl.

Preferably at least three of K', K", K"' and K"" are -CH-; more preferably K', K", K"' and K"" are all -CH-. When one of the K's are -CR⁴- or -CR⁵-, then R⁴ or R⁵ is preferably halogen.

In the various "L" substituents, it is preferred that R³³, R³⁴, R³⁵ and R³⁶ are independently selected from the group consisting of H and C₁₋₆alkyl; and are more preferably independently selected from the group consisting of H and methyl. It is also preferred that R³⁷ is H, C₁₋₆alkyl or taken together with R³⁵ or R³⁶ to form a 5-6 membered ring; and is more preferably H or methyl. It is also preferred that R³⁸ is H, C₁₋₆alkyl or taken together with R³⁶ to form a 5-6 membered ring; and is more preferably H or methyl. M is preferably a direct link, C₁₋₆alkyl, C₃₋₈cycloalkyl, aryl, or a five to ten membered heterocyclic ring system. More preferably, M is C₁₋₄alkyl, aryl, or a five to ten membered heterocyclic ring system. The integer q is preferably 0.

Q is preferably: 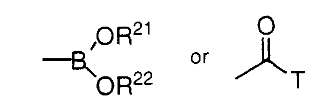 where R²¹ is preferably H and R²² is preferably H.

T is preferably H, -COOR²³, -CONR²³R²⁴ or a group having the formula: 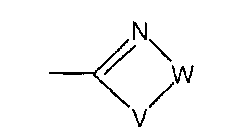 R²³ is preferably H. R²⁴ is preferably C₁₋₄alkylaryl. V is preferably -S-, -O- or -NR²⁶-, where R²⁶ is preferably H or methyl, more preferably H. W is preferably selected from the group consisting of: 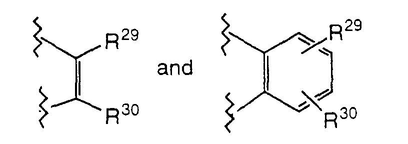 W is more preferably 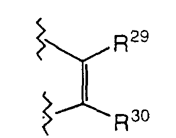 R²⁹ and R³⁰ are preferably independently selected from the group consisting of H, -O-R³¹, -COOR³¹, -CONR³¹R³² or -CF₃; more preferably H.

When W is: 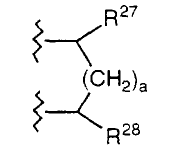 then R²⁷ is preferably H and R²⁸ is preferably H.

When T is: 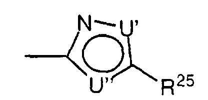 then U' is preferably O, U" is preferably N and R²⁵ is preferably -CF₃ or -CF₂CF₃.

In one preferred embodiment of the invention, s is 0; R² and R³ are H; K'. K". K"' and K"" are -CH-; and Q is -C(O)-T. This is also illustrated as a preferred group of compounds defined by the general structural formula II as:

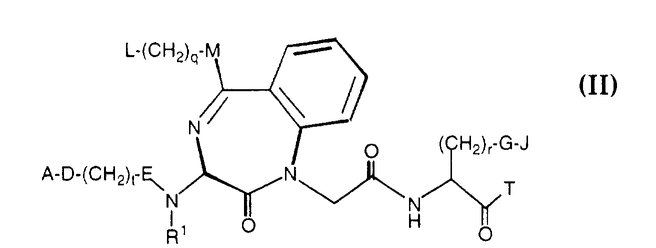

A preferred embodiment of compounds of general structural formula II have the following stereochemistry:

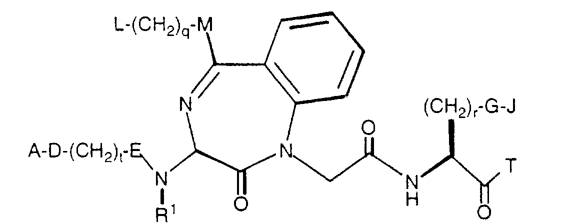

In another preferred embodiment of the invention, s is 0; r is 3; R¹, R² and R³ are H; K'. K". K"' and K"" are -CH-; G is a bond; J is: 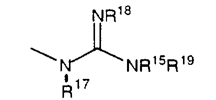 where R¹⁵, R¹⁷, R¹⁸ and R¹⁹ are all H; and Q is -C(O)-T. This is also illustrated as a preferred group of compounds defined by the general structural formula III as:

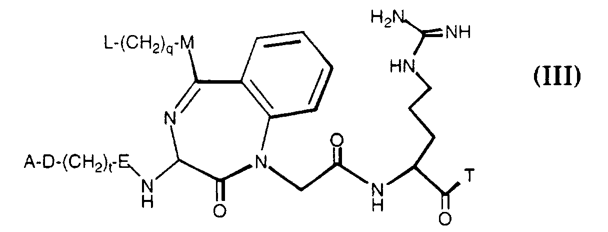

A preferred embodiment of compounds of general structural formula III have the following stereochemistry:

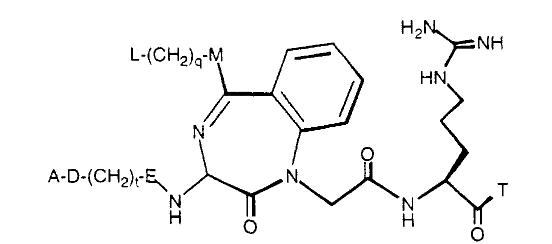

This invention also encompasses all pharmaceutically acceptable isomers. salts, hydrates and solvates of the compounds of formulas I, II and III. In addition, the compounds of formulas I, II and III can exist in various isomeric and tautomeric forms, and all such forms are meant to be included in the invention, along with pharmaceutically acceptable salts, hydrates and solvates of such isomers and tautomers.

The compounds of this invention may be isolated as the free acid or base or converted to salts of various inorganic and organic acids and bases. Such salts are within the scope of this invention. Non-toxic and physiologically compatible salts are particularly useful although other less desirable salts may have use in the processes of isolation and purification.

A number of methods are useful for the preparation of the salts described above and are known to those skilled in the art. For example, the free acid or free base form of a compound of one of the formulas above can be reacted with one or more molar equivalents of the desired acid or base in a solvent or solvent mixture in which the salt is insoluble, or in a solvent like water after which the solvent is removed by evaporation, distillation or freeze drying. Alternatively, the free acid or base form of the product may be passed over an ion exchange resin to form the desired salt or one salt form of the product may be converted to another using the same general process.

This invention also encompasses prodrug derivatives of the compounds contained herein. The term "prodrug" refers to a pharmacologically inactive derivative of a parent drug molecule that requires biotransformation, either spontaneous or enzymatic, within the organism to release the active drug. Prodrugs are variations or derivatives of the compounds of this invention which have groups cleavable under metabolic conditions. Prodrugs become the compounds of the invention which are pharmaceutically active *in vivo,* when they undergo solvolysis under physiological conditions or undergo enzymatic degradation. Prodrug compounds of this invention may be called single, double, triple etc., depending on the number of biotransformation steps required to release the active drug within the organism, and indicating the number of functionalities present in a precursor-type form. Prodrug forms often offer advantages of solubility, tissue compatibility, or delayed release in the mammalian organism (see, Bundgard, Design of Prodrugs, pp. 7-9, 21-24, Elsevier, Amsterdam 1985 and Silverman, The Organic Chemistry of Drug Design and Drug Action, pp. 352-401, Academic Press, San Diego, CA, 1992). Prodrugs commonly known in the art include acid derivatives well known to practitioners of the art, such as, for example, esters prepared by reaction of the parent acids with a suitable alcohol, or amides prepared by reaction of the parent acid compound with an amine, or basic groups reacted to form an acylated base derivative. Moreover, the prodrug derivatives of this invention may be combined with other features herein taught to enhance bioavailability.

The following structures are illustrative of the compounds of the present invention and are not intended to be limiting in any manner. It is to be noted that in the compounds of the invention, certain substituents are present between two other substituents. For example, D is positioned between A- and -(CH₂)ₜ-E-. Accordingly, substituents such as D are illustrated below as having two "dangling" bonds, the bond on the left representing a direct link to substituent A- and the bond on the right representing a direct link to -(CH₂)ₜ-E-. Therefore, the general formula of A-D-(CH₂)ₜ-E- where D is phenyl can be written as: 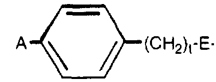 D, a phenyl group, would then be written as follows in the tables below: 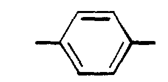 Other substituents in the table below may also be presented as having one or two similar "dangling" bonds. It is understood that these represent direct links to the adjacent substituent(s). It is also understood that the compounds illustrated below can exist as other isomers, and the isomeric form illustrated herein is not intended to be limiting in any manner.

The invention encompasses compounds of general structural formula IV, where R¹, R² and R3 are H; r is 3; q and s are 0; G is a direct link; J is -NH-C(NH)NH₂; K', K", K"' and 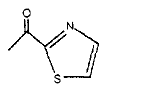 K"" are -CH-; L is H; M is a direct link; and Q is

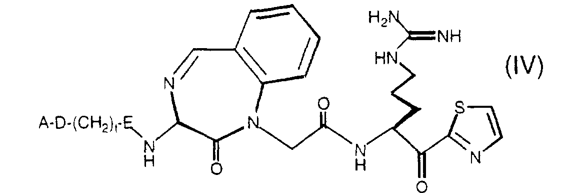

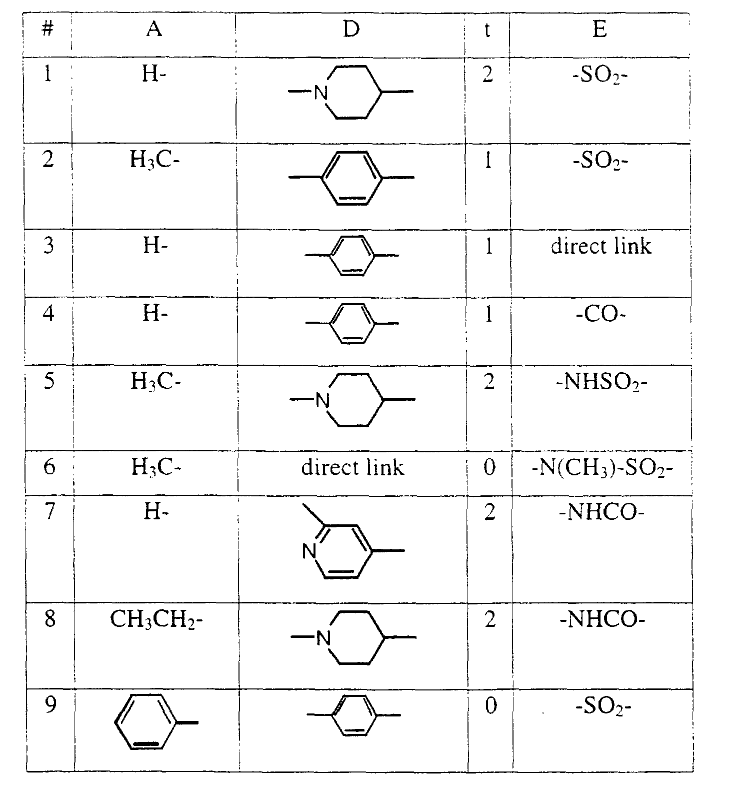

The invention encompasses compounds of general structural formula V, where R¹, R² and R³ are H; r is 3; q and s are 0; G is a direct link; J is -NH-C(NH)NH₂; K', K", K"' and K"" are -CH-; L is H; M is

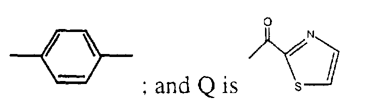

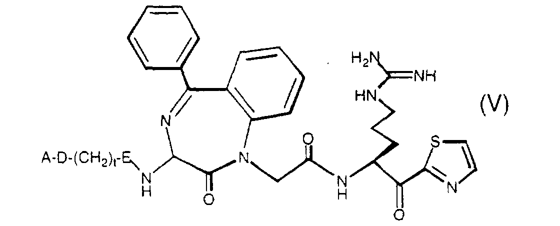

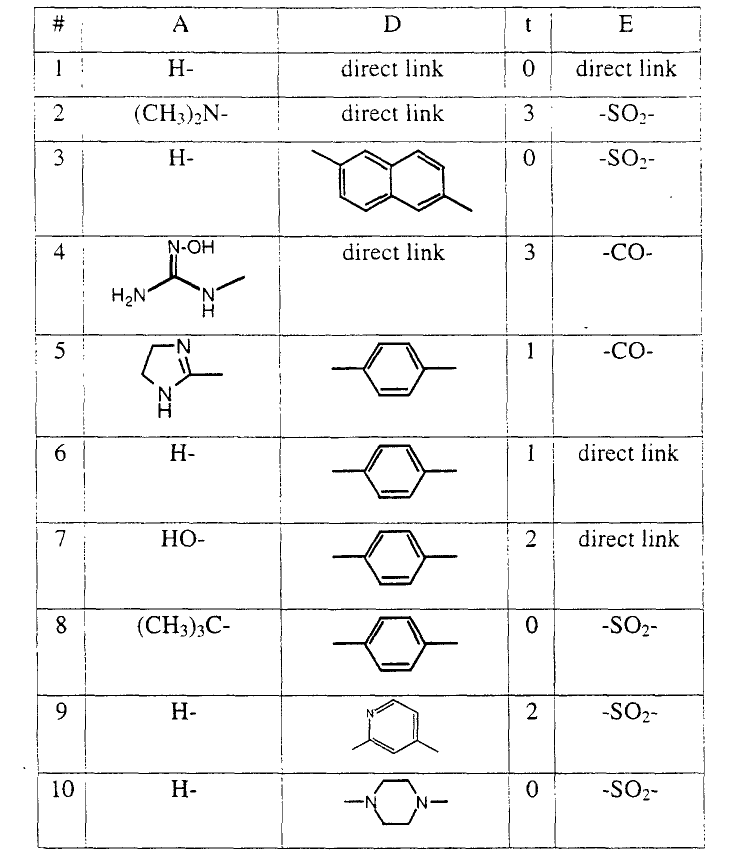

The invention encompasses compounds of general structural formula VI, where R¹, R² and R³ are H; q is 0; t is 1; A is H; D is phenyl; E is -SO₂-; K', K", K"' and K"" are -CH-; L is H; M is a direct link; and Q is

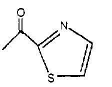

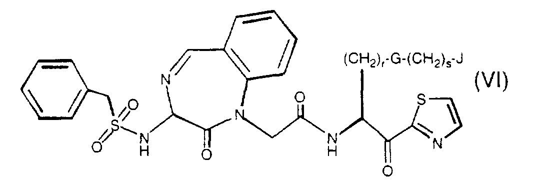

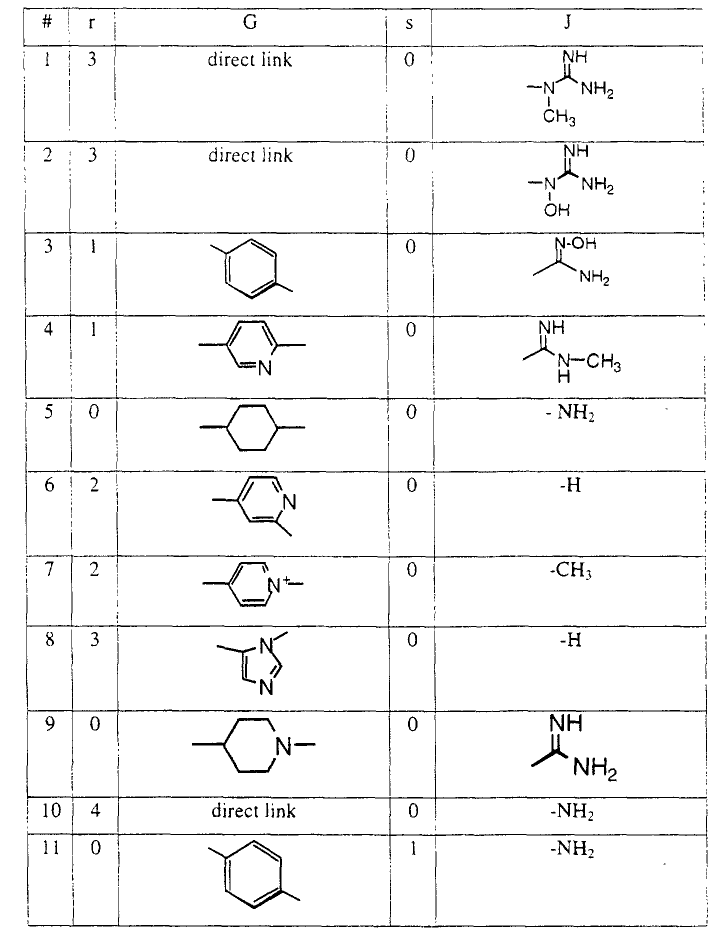

The invention encompasses compounds of general structural formula VII, where q is 0; r is 3; s is 0; t is 1; A is H; D is phenyl; E is -SO₂-; G is a direct link; J is -NH- C(NH)NH₂; K', K", K"' and K"" are -CH-; L is H; M is a direct link; and Q is

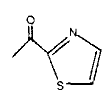

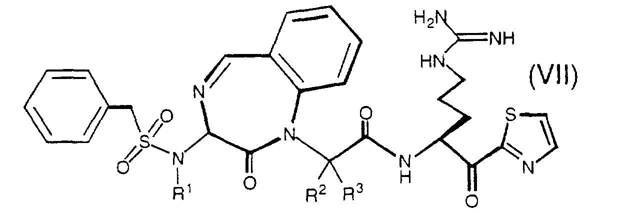

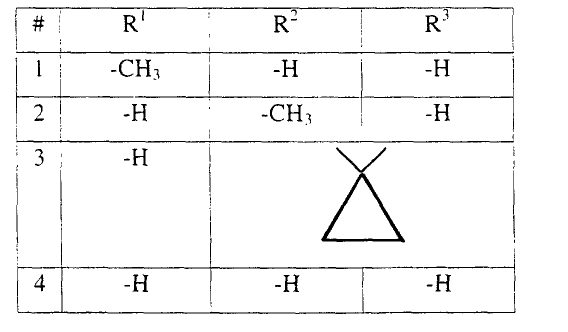

The invention encompasses compounds of general structural formula VIII, where R', R² and R³ are H; q is 0; r is 3; s is 0; t is 1; A is H; D is phenyl; E is -SO₂-; G is a direct link; J is -NH-C(NH)NH₂; K', K", K"' and K"" are -CH-; L is H; and M is a direct link:

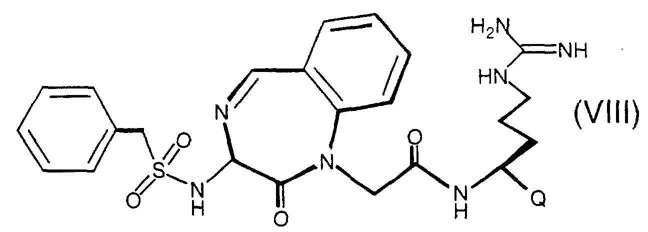

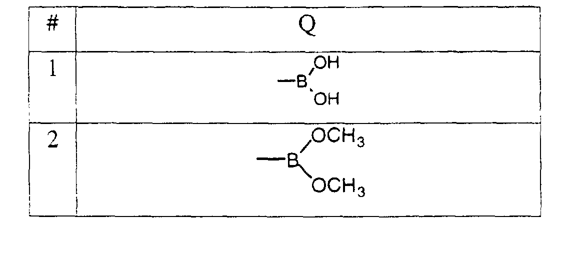

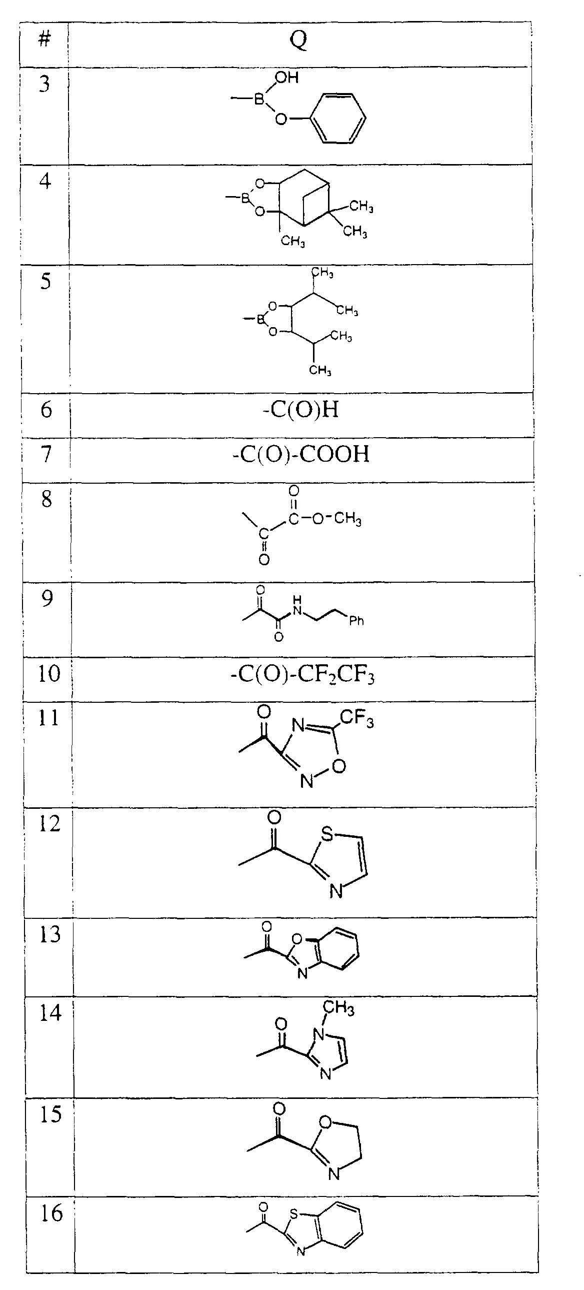

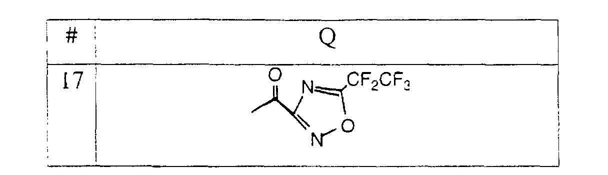

The invention encompasses compounds of general structural formula IX, where R¹, R² and R³ are H; q and s are 0; r is 3; t is 1; A and L are H; D is phenyl; E is -SO₂-; G and M are direct links; J is -NH-C(NH)NH₂; and Q is

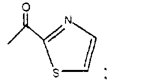

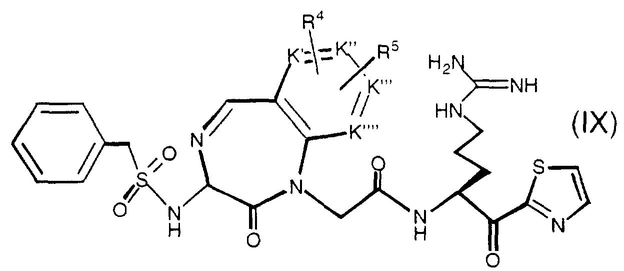

| # | K' | K" | K"' | K"" |
|---|---|---|---|---|
| 1 | -CH- | -N- | -CH- | -CH- |
| 2 | -C(Cl)- | -N- | -CH- | -CH- |

The invention encompasses compounds of general structural formula X, where R¹, R² and R³ are H; s is 0; r is 3; t is 1; A is H; D is phenyl; E is -SO₂-; G is a direct link; K', K", K"' and K"" are -CH-, J is -NH-C(NH)NH₂; and Q is

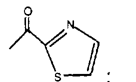

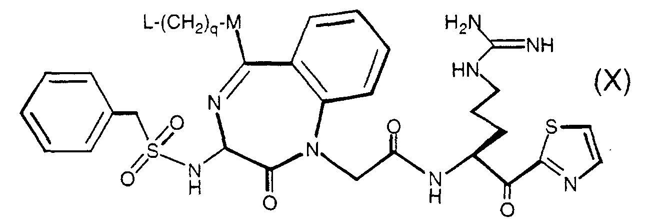

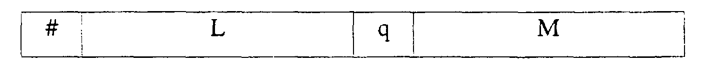

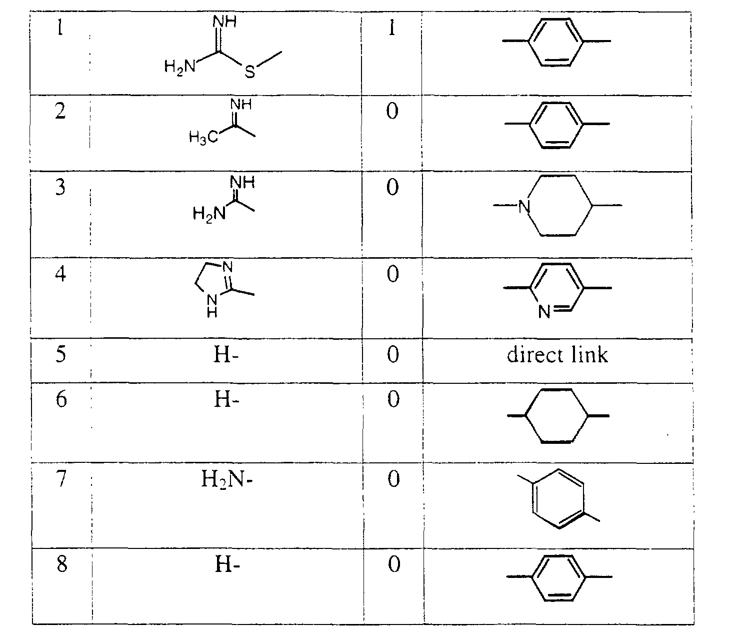

As mentioned above, the compounds of this invention find utility as therapeutic agents for disease states in mammals which have disorders of coagulation such as in the treatment or prevention of unstable angina, refractory angina, myocardial infarction, transient ischemic attacks, thrombotic stroke, embolic stroke, disseminated intravascular coagulation including the treatment of septic shock, deep venous thrombosis in the prevention of pulmonary embolism or the treatment of reocclusion or restenosis of reperfused coronary arteries. Further, these compounds are useful for the treatment or prophylaxis of those diseases which involve the production and/or action of factor Xa/prothrombinase complex. This includes a number of thrombotic and prothrombotic states in which the coagulation cascade is activated which include but are not limited to, deep venous thrombosis, pulmonary embolism, myocardial infarction, stroke, thromboembolic complications of surgery and peripheral arterial occlusion.

Accordingly, a method for preventing or treating a condition in a mammal characterized by undesired thrombosis comprises administering to the mammal a therapeutically effective amount of a compound of this invention. In addition to the disease states noted above, other diseases treatable or preventable by the administration of compounds of this invention include, without limitation, occlusive coronary thrombus formation resulting from either thrombolytic therapy or percutaneous transluminal coronary angioplasty, thrombus formation in the venous vasculature, disseminated intravascular coagulopathy, a condition wherein there is rapid consumption of coagulation factors and systemic coagulation which results in the formation of life-threatening thrombi occurring throughout the microvasculature leading to widespread organ failure, hemorrhagic stroke, renal dialysis, blood oxygenation, and cardiac catheterization.

The compounds of the invention also find utility in a method for inhibiting the coagulation biological samples, which comprises the administration of a compound of the invention.

The compounds of the present invention may also be used in combination with other therapeutic or diagnostic agents. In certain preferred embodiments, the compounds of this invention may be coadministered along with other compounds typically prescribed for these conditions according to generally accepted medical practice such as anticoagulant agents, thrombolytic agents, or other antithrombotics, including platelet aggregation inhibitors, tissue plasminogen activators, urokinase, prourokinase, streptokinase, heparin. aspirin, or warfarin. The compounds of the present invention may act in a synergistic fashion to prevent reocclusion following a successful thrombolytic therapy and/or reduce the time to reperfusion. These compounds may also allow for reduced doses of the thrombolytic agents to be used and therefore minimize potential hemorrhagic side-effects. The compounds of this invention can be utilized *in vivo*, ordinarily in mammals such as primates, (e.g. humans), sheep, horses, cattle, pigs, dogs, cats, rats and mice, or *in vitro.*

The biological properties of the compounds of the present invention can be readily characterized by methods that are well known in the art, for example by the *in vitro* protease activity assays and *in vivo* studies to evaluate antithrombotic efficacy, and effects on hemostasis and hematological parameters, such as are illustrated in the examples.

Diagnostic applications of the compounds of this invention will typically utilize formulations in the form of solutions or suspensions. In the management of thrombotic disorders the compounds of this invention may be utilized in compositions such as tablets, capsules or elixirs for oral administration, suppositories, sterile solutions or suspensions or injectable administration, and the like, or incorporated into shaped articles. Subjects in need of treatment (typically mammalian) using the compounds of this invention can be administered dosages that will provide optimal efficacy. The dose and method of administration will vary from subject to subject and be dependent upon such factors as the type of mammal being treated, its sex, weight, diet, concurrent medication, overall clinical condition, the particular compounds employed, the specific use for which these compounds are employed, and other factors which those skilled in the medical arts will recognize.

Formulations of the compounds of this invention are prepared for storage or administration by mixing the compound having a desired degree of purity with physiologically acceptable carriers, excipients, stabilizers etc., and may be provided in sustained release or timed release formulations. Acceptable carriers or diluents for therapeutic use are well known in the pharmaceutical field, and are described, for example, in Remington's Pharmaceutical Sciences, Mack Publishing Co., (A.R. Gennaro edit. 1985). Such materials are nontoxic to the recipients at the dosages and concentrations employed, and include buffers such as phosphate, citrate, acetate and other organic acid salts, antioxidants such as ascorbic acid, low molecular weight (less than about ten residues) peptides such as polyarginine, proteins, such as serum albumin, gelatin, or immunoglobulins, hydrophilic polymers such as polyvinylpyrrolidinone, amino acids such as glycine, glutamic acid, aspartic acid, or arginine, monosaccharides, disaccharides, and other carbohydrates including cellulose or its derivatives, glucose, mannose or dextrins, chelating agents such as EDTA, sugar alcohols such as mannitol or sorbitol, counterions such as sodium and/or nonionic surfactants such as Tween, Pluronics or polyethyleneglycol.

Dosage formulations of the compounds of this invention to be used for therapeutic administration must be sterile. Sterility is readily accomplished by filtration through sterile membranes such as 0.2 micron membranes, or by other conventional methods. Formulations typically will be stored in lyophilized form or as an aqueous solution. The pH of the preparations of this invention typically will be 3-11, more preferably 5-9 and most preferably 7-8. It will be understood that use of certain of the foregoing excipients, carriers, or stabilizers will result in the formation of cyclic polypeptide salts. While the preferred route of administration is by injection, other methods of administration are also anticipated such as orally, intravenously (bolus and/or infusion), subcutaneously, intramuscularly, colonically, rectally, nasally, transdermally or intraperitoneally, employing a variety of dosage forms such as suppositories, implanted pellets or small cylinders, aerosols, microencapsulation, oral dosage formulations and topical formulations such as ointments, drops and dermal patches. The compounds of this invention are desirably incorporated into shaped articles such as implants which may employ inert materials such as biodegradable polymers or synthetic silicones, for example, Silastic, silicone rubber or other polymers commercially available.

The compounds of the invention may also be administered in the form of liposome delivery systems, such as small unilamellar vesicles, large unilamellar vesicles and multilamellar vesicles. Liposomes can be formed from a variety of lipids, such as cholesterol, stearylamine or phosphatidylcholines.

The compounds of this invention may also be delivered by the use of antibodies, antibody fragments, growth factors, hormones, or other targeting moieties, to which the compound molecules are coupled. The compounds of this invention may also be coupled with suitable polymers as targetable drug carriers. Such polymers can include polyvinylpyrrolidinone, pyran copolymer, polyhydroxy-propyl-methacrylamide-phenol, polyhydroxyethyl-aspartamide-phenol, or polyethyleneoxide-polylysine substituted with palmitoyl residues. Furthermore, compounds of the invention may be coupled to a class of biodegradable polymers useful in achieving controlled release of a drug, for example polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels. Polymers and semipermeable polymer matrices may be formed into shaped articles, such as valves, stems, tubing, prostheses and the like.

Therapeutic compound liquid formulations generally are placed into a container having a sterile access port, for example, an intravenous solution bag or vial having a stopper pierceable by hypodermic injection needle.

Therapeutically effective dosages may be determined by either *in vitro* or *in vivo* methods. For each particular compound of the present invention, individual determinations may be made to determine the optimal dosage required. The range of therapeutically effective dosages will be influenced by the route of administration, the therapeutic objectives and the condition of the patient. For injection by hypodermic needle, it may be assumed the dosage is delivered into the body's fluids. For other routes of administration, the absorption efficiency must be individually determined for each compound by methods well known in pharmacology. Accordingly, it may be necessary for the therapist to titer the dosage and modify the route of administration as required to obtain the optimal therapeutic effect. The determination of effective dosage levels, that is, the dosage levels necessary to achieve the desired result, will be readily determined by one skilled in the art. Typically, applications of compound are commenced at lower dosage levels, with dosage levels being increased until the desired effect is achieved.

The compounds of the invention can be administered orally or parenterally in an effective amount within the dosage range of about 0.1 to 100 mg/kg, preferably about 0.5 to 50 mg/kg and more preferably about 1 to 20 mg/kg on a regimen in a single or 2 to 4 divided daily doses and/or continuous infusion.

Typically, about 5 to 500 mg of a compound or mixture of compounds of this invention, as the free acid or base form or as a pharmaceutically acceptable salt, is compounded with a physiologically acceptable vehicle, carrier, excipient, binder, preservative, stabilizer, dye, flavor etc., as called for by accepted pharmaceutical practice. The amount of active ingredient in these compositions is such that a suitable dosage in the range indicated is obtained.

Typical adjuvants which may be incorporated into tablets, capsules and the like are binders such as acacia, corn starch or gelatin, and excipients such as microcrystalline cellulose, disintegrating agents like corn starch or alginic acid, lubricants such as magnesium stearate, sweetening agents such as sucrose or lactose, or flavoring agents. When a dosage form is a capsule, in addition to the above materials it may also contain liquid carriers such as water, saline, or a fatty oil. Other materials of various types may be used as coatings or as modifiers of the physical form of the dosage unit. Sterile compositions for injection can be formulated according to conventional pharmaceutical practice. For example, dissolution or suspension of the active compound in a vehicle such as an oil or a synthetic fatty vehicle like ethyl oleate, or into a liposome may be desired. Buffers, preservatives, antioxidants and the like can be incorporated according to accepted pharmaceutical practice.

### Preparation of the Disclosed Compounds

The compounds of the present invention may be synthesized by either solid or liquid phase methods described and referenced in standard textbooks, or by a combination of both methods. These methods are well known in the art. See, Bodanszky, "The Principles of Peptide Synthesis", Hafner, *et al*., Eds., Springer-Verlag, Berlin, 1984.

Starting materials used in any of these methods are commercially available from chemical vendors such as Aldrich, Sigma, Nova Biochemicals, Bachem Biosciences, and the like, or may be readily synthesized by known procedures.

Reactions are carried out in standard laboratory glassware and reaction vessels under reaction conditions of standard temperature and pressure, except where otherwise indicated. The reaction products are isolated and purified by conventional methods, typically by solvent extraction into a compatible solvent. The products may be further purified by column chromatography or other appropriate methods. Most compounds are purified by reversed-phase HPLC and characterized by ion-spray mass spectrometry.

During the synthesis of these compounds, the functional groups of the amino acid derivatives used in these methods are protected by blocking groups to prevent side reactions during the coupling procedure. Examples of suitable blocking groups and their use are described in "The Peptides: Analysis, Synthesis, Biology", Academic Press, Vol. 3 (Gross, *et al*., Eds., 1981) and Vol. 9 (1987).

The compounds of this invention may be preferably prepared by coupling the carboxylic acid of formula (a) to the amine of formula (b) by the standard amide bond formation strategies:

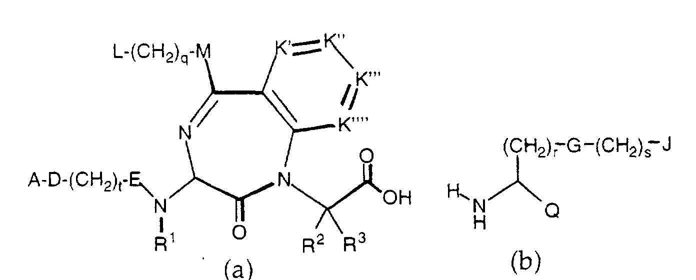

The compounds of formula (b) wherein Q is H can be prepared by the methods disclosed in WO 96/01338; WO 96/24609: Feng, *et al*., WO 96/31504; and WO 96/32110.

The compounds of formula (b) wherein Q is a boron containing compound can be prepared by the methods disclosed in J. Org. Chem. 60:3717-3722 (1995) and de Nanteuil, *et al*., EP 688,788.

The compounds of formula (b) wherein Q is -C(O)-T, where T is H, may be prepared by the methods disclosed in WO 93/15756, *supra*; Vlasuk, *et al*., WO 94/17817; Abelman, *et al*., WO 94/21673; Webb, *et al*., WO 94/08941; Veber, *et al*., WO 94/25051; Levy, *et al*., WO 95/35312; Semple, *et al*., WO 95/35313; Abelman, *et al*., WO 95/28420; and Abelman, *et al*., WO 96/19493.

The compounds of formula (b) wherein Q is -C(O)-T, where T is -COOR²³ or -CONR²³R²⁴, may be prepared by the methods disclosed in WO 94/25051, *supra*, WO 94/08941, *supra*, and WO 94/21673, *supra.*

The compounds of formula (b) wherein Q is -C(O)-T, where T is -CF₃ or -CF₂CF₃, may be prepared by the methods disclosed in Schacht, *et al*., GB 2287027.

The compounds of formula (b) wherein Q is -C(O)-T, where T is: 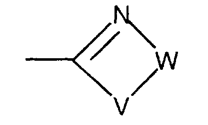 and V is -S-, -O-, -SO- or -SO₂- can be readily synthesized by the methods disclosed in Costanzo, *et al*., U.S. Pat. No. 5,523,308; Di Maio, *et al.*, WO 96/19483; U.S. Pat. No. 5,164,371; J. Am. Chem. Soc. 114: 1854-1863 (1992); J. Med. Chem. 38:76-85 (1995); and J. Med. Chem. 37:3492-3502 (1994). Lastly, fragments where V is -NR²¹-, where R²¹ is H, C₁₋₆alkyl or benzyl, can be synthesized by techniques illustrated in J. Med. Chem. 37:3492-3502 (1994).

The compounds of formula (b) wherein Q is -C(O)-T, where T is: 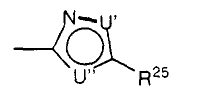 and U' and U" are the various substituents (-O-, -S-, -N-, -NH-) may be prepared by the methods disclosed in J. Med. Chem. 38: 1355-1371 (1995) and J. Med. Chem. 37: 2421-2436 (1994).

The starting compound of formula (a) is either a known compound or can be produced by known methods (Heitsch, *et al*., Canadian Patent No. 2,071,744; Sugihara, *et al*., Canadian Patent No. 2,126,026; Baker, *et al*., EP 365,992; U.S. Pat. No. 4,251,438; Carr, *et al*., U.S. Pat. No. 4,341,698; Goldman, *et al*., U.S. Pat. No. 5,120,718; Biswanath, *et al*., U.S. Pat. No. 5.164,388; Duggan, *et al*., *U.S.* Pat. No. 5,281,585; Sugihara, *et al*., U.S. Pat. No. 5,294,713; Bovy, *et al*., WO 95/06038; WO 95/35308; J. Chem. Soc. Perkin Trans. I 1687-1689 (1989); and Int. J. Peptide Protein Res. 37:468-475 (1991)) or can be prepared by the methods shown in the following reaction formulae.

The following reaction schemes are more specific illustrations of the above reaction formulae. The chemical reactions described in each scheme can easily be modified and combined with other techniques that are well known in the art to produce other compounds within the scope of the invention.

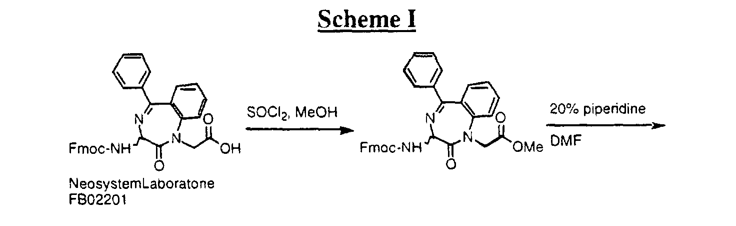

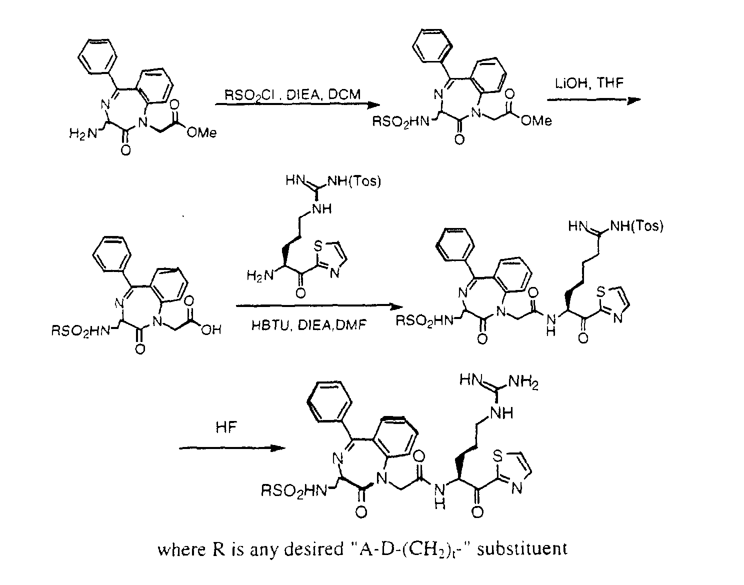

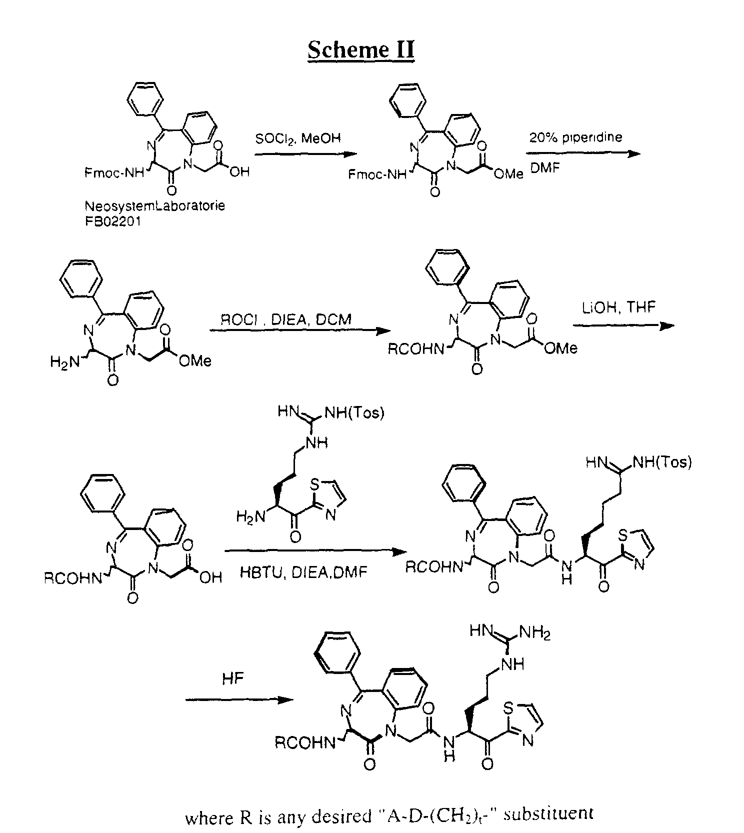

The reagent used in the fifth step of Schemes I and II can be synthesized as follows:

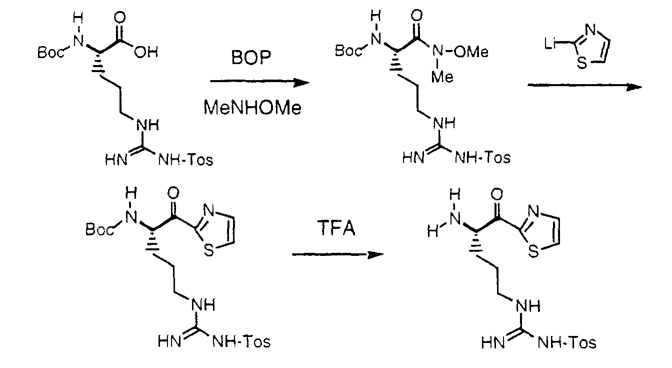

Without further elaboration, it is believed that one skilled in the art can utilize the present invention to its fullest extent. Therefore, the following preferred specific embodiments are to be construed as merely illustrative and do not limit the remainder of the disclosure in any way whatsoever.

### Example 1

Preparation of:

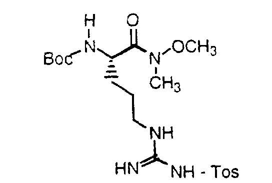

To a suspension of Boc-Arg(Tos)-OH (2 g, 4.7 mmol) in DMF (20 mL) at 0°C was added MeNHOMe•HCl (1 g, 10.3 mmol), DIEA (6 mL) and BOP (2.5 g, 5.6 mmol). The solution was stirred at 0°C for 10 hours. DMF was evaporated by vacuum. The oily residue was dissolved in EtOAc (200 mL) and water (20 mL). The organic layer was washed with sat. NaHCO₃, water (20 mL), 1 M HCl (10 mL) and sat. NaCl (2 X 20 mL). The organic layer was dried over MgSO₄, filtered and evaporated to give a suspension. The suspension was filtered, and the solid was washed with cold EtOAc (10 mL) and dried to give Boc-Arg(Tos)-N(Me)OMe, shown above, (1.5 g, 70 % yield).
FAB-MS (M+H)+ = 472

### Example 2

Preparation of:

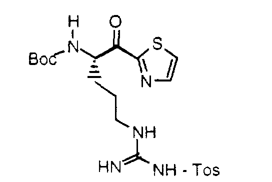

To a solution of thiazole (2.5 g, 29 mmol) in THF (25 mL) at -78°C was added n-BuLi (1.6 M in hexane, 19 mL) dropwise. The mixture was stirred for 30 minutes. Then a solution of Boc-Arg(Tos)-N(Me)OMe, from Example 1, (1.7 g, 3.6 mmol) in THF (50 mL) was added to the lithiothiazole mixture at -78°C. The solution was stirred for 2 hours. 1M HCI (30 mL) was added to the reaction mixture and warmed to room temperature. The mixture was extracted with EtOAc (100 mL). The organic layer was washed with sat. NaCl (30 mL), dried over MgSO₄, filtered and evaporated. The crude oily residue was purified by flash column chromatography over SiO₂ (50% EtOAc in CH₂Cl₂) to give Boc-Arg(Tos)-thiazole, shown above, (1.5 g, 84% yield) as a powder. DCI -MS (M+H)+ = 496

### Example 3

Preparation of:

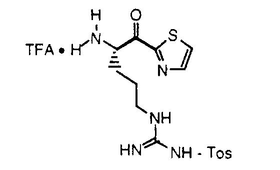

To a solution of Boc-Arg(Tos)-thiazole from Example 2, (300 mg, 0.6 mmol) in CH₂Cl₂ (10 mL) at 0°C, was added TFA (10 mL). The solution was stirred at 0°C for 2 hours. The solvent and excess TFA were evaporated to give an oily residue which was then used directly without further purification.

### Example 4

### Part 1 Preparation of methyl 2-(3-(((9H-9-fluorenylmethoxy)carbonyl)amino)-2-oxo-5-phenyl-2,3-dihydro- 1H-1,4-benzodiazepin- 1-yl)acetate:

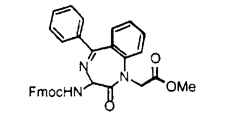

A solution of (R,S)Fmoc-3-amino-N-1-carboxymethyl-2-oxo-5-phenyl-1,4-benzodiazepine from Neosystem Laboratorie (Strasbourg, France) (212 mg, 0.4 mmol) in MeOH at 0°C, was treated with excess amount of thionyl chloride (20 eq). The mixture was stirred at room temperature overnight and evaporated to give the title compound (100% yield). ES-MS (M+H)+ = 546.2.

### Part 2 Preparation of methyl 2-(3-amino-2-oxo-5-phenyl-2,3-dihydro-1H-1,4-benzodiazepin-1-yl)acetate:

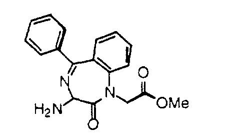

A solution of the compound of Part 1 (226 mg, 0.4 mmol) in DMF (2mL) was treated with piperidine (0.5 mL, 5 mmol) to make a final solution of 20% piperidine/DMF and stirred at room temperature overnight. The reaction mixture was evaporated *in vacuo* then acidified with 10% HCl and extracted with CH₂Cl₂. The aqueous layer was then neutralized with IN NaOH and extracted with EtOAc. The organic layer was dried over Na₂SO₄ and evaporated *in vacuo* to give title compound (99% yield). ES-MS (M+H)+ = 324.2.

### Part 3 Preparation of methyl 2-(3-((benzylsulfonyl)amino)-2-oxo-5-phenyl-2,3-dihydro-1H-1,4-benzodiazepin-1-yl)acetate:

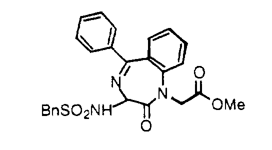

To a solution of the compound of Part 2 (128 mg, 0. 4 mmol) and DIEA (0.092 mL, 0.5 mmol) in CH₂Cl₂ (2 mL) at -78 °C, was added α-toluenesulfonyl chloride (92 mg, 0.48 mmol). The mixture was stirred at -78 °C for three hours and evaporated *in vacuo*. The residue was dissolved in EtOAc and washed with H₂O, sat. NaHCO₃, sat. NaCl, dried over Na₂SO₄ and evaporated to give the title compound as a solid (67% yield). ES-MS (M+H)+ = 478.3.

### Part 4 Preparation of 2-(3-((benzylsulfonyl)amino)-2-oxo-5-phenyl-2,3-dihydro-1H-1,4-benzodiazepin-1-yl)acetic acid:

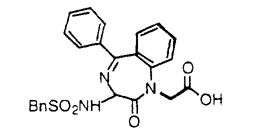

A solution of the compound of Part 3 (128 mg, 0.27 mmol) in THF (2 mL) was treated with 2 N LiOH (0.54 mL, 1.08 mmol). The mixture was stirred at room temperature overnight. The organic solvent was evaporated *in vacuo*. The aqueous layer was acidified to pH 2-3 with IN aq HCl, extracted with EtOAc and the organic layer was dried over Na₂SO₄ and evaporated to give the title compound (100% yield). ES-MS (M+H)+ = 464.0.

### Part 5 Preparation of N1-(1R)-4-((imino(((4-methylphenyl)sulfonyl)amino)methyl) amino)-1-(1,3-thiazol-2-ylcarbonyl)butyl)-2-(3-((benzylsulfonyl)amino)-2-oxo-5-phenyl-2,3-dihydro- 1H-1,4-benzodiazepin-1-yl)acetamide:

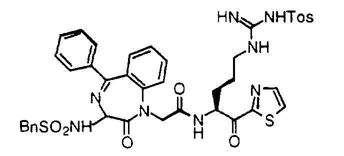

The compound of Part 4 (0.3 mmol) was dissolved in DMF (2 mL) and cooled to 0°C. The solution was neutralized with DIEA followed by addition of the compound of Example 3 (142 mg, 0.36 mmol) and coupling reagent HBTU (136 mg, 0.36 mmol). The solution was stirred at room temperature overnight. The reaction mixture was diluted in a mixture of EtOAc/H₂O. The organic layer was washed with sat. NaHCO₃, sat. NaCI, dried over Na₂SO₄, and solvent evaporated to give the title compound (82% yield). ES-MS (M+H)+ = 841.4.

### Part 6 Preparation of N1-((1R)-4-((amino(imino)methyl)amino)-1-(1,3-thiazol-2-ylcarbonyl)butyl)-2-(3-((benzylsulfonyl)amino)-2-oxo-5-phenyl-2,3-dihydro-1H-1,4-benzodiazepin-1-yl)acetamide (Compound X(8)):

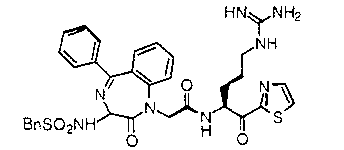

The compound of Part 5 (206 mg, 0.25 mmol), anisole (1 mL) and ethyl methyl sulfide (two drops) were placed in HF-cleavage vessel and cooled under liquid N₂. HF (10 mL) was then condensed and the mixture was stirred at -10°C for half hour and 0°C for half hour. HF was removed under vacuum to give a gum-like residue. The residue was triturated with 50% Et₂O in hexane (20 mL) and the solvent removed by filtration. The gum residue was dissolved in 0.1% aq. TFA (15 mL) and filtered through the above sintered funnel. The filtrate was lyophilized to give a powder which was purified by RP-HPLC to give the title compound as inseparable diastereoisomers as a white powder (85% yield). ES-MS (M+H)+ = 686.8.

### Example 5

### Part 1 Preparation of 9H-9-fluorenylmethyl N-(1-(2-(((1R)-4-((imino(((4-methylphenyl)sulfonyl)amino)methyl)amino)-1-(1,3-thiazol-2-ylcarbonyl)butyl)amino)-2-oxoethyl)-2-oxo-5-phenyl-2,3-dihydro-1H-1,4-benzodiazepin-3-yl)carbamate

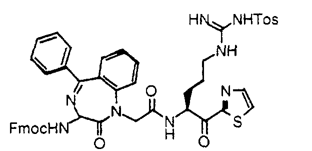

(R,S)Fmoc-3-amino-N-1-carboxymethyl-2-oxo-5-phenyl-1,4-benzodiazepine from Neosystem Laboratorie (134 mg, 0.25 mmol) was dissolved in DMF (2 mL) and cooled to 0°C. The solution was neutralized with DIEA followed by addition of the compound of Example 3 (118 mg, 0.3 mmol) and coupling reagent HBTU (114 mg, 0.3 mmol). The solution was stirred overnight at room temperature. The reaction mixture was diluted in a mixture of EtOAc/H₂O. The organic layer was washed with sat. NaHCO₃, sat. NaCl, dried over Na₂SO₄, filtered and solvent evaporated to give the title compound (93% yield). ES-MS (M+H)+ = 909.3.

### Part 2 Preparation of N1-((1R)-4-((imino(((4-methylphenyl)sulfonyl)amino)methyl) amino)-1-(1,3-thiazol-2-ylcarbonyl)butyl)-2-(3-amino-2-oxo-5-phenyl-2,3-dihydro-1H-1,4-benzodiazepin-1-yl)acetamide:

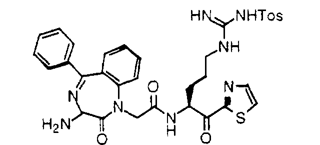

A solution of the compound of Part 1 (211 mg, 0.232 mmol) in DMF (2 mL) was treated with piperidine (0.5 mL, 5 mmol) to make a final solution of 20% piperidine/DMF and stirred at room temperature overnight. The reaction mixture was evaporated *in vacuo* then acidified with 10% HCl and extracted with CH₂Cl₂. The aqueous layer was then neutralized with IN NaOH and extracted with EtOAc. The organic layer was dried over Na₂SO₄ and evaporated *in vacuo* to give title compound (100% yield). ES-MS (M+H)+ = 687.1.

### Part 3 Preparation of N1-((1R)-4-((amino(imino)methyl)amino)-1-(1,3-thiazol-2-ylcarbonyl)butyl)-2-(3-amino-2-oxo-5-phenyl-2,3-dihydro-1H-1,4-benzodiazepin-1-yl)acetamide (Compound V(1)):

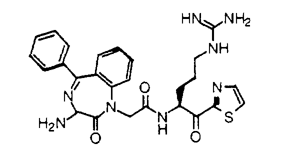

The compound of Part 2 (0.232 mmol), anisole (1 mL) and ethyl methyl sulfide (two drops) were placed in HF-cleavage vessel and cooled under liquid N2. HF (10 mL) was then condensed and the mixture was stirred at -10°C for half hour and 0°C for half hour. HF was removed under vacuum to give a gum-like residue. The residue was triturated with 50% Et₂O in hexane (20 mL) and the solvent removed by filtration. The gum residue was dissolved in 0.1% aq. TFA (15 mL) and filtered through the above sintered funnel. The filtrate was lyophilized to give a powder which was purified by RP-HPLC to give the title compound as inseparable diastereoisomers as a white powder (88% yield). ES-MS (M+H)+ = 533.1.

### Example 6

### (Determination of IC₅₀)

The compounds of the present invention are first dissolved in a buffer to give solutions containing concentrations such that assay concentrations range from 0-100 µM. In assays for thrombin, prothrombinase and factor Xa, a synthetic chromogenic substrate would be added to a solution containing a test compound and the enzyme of interest and the residual catalytic activity of that enzyme would then be determined spectrophotometrically.

The IC₅₀ of a compound is determined from the substrate turnover. The IC₅₀ is the concentration of test compound giving 50% inhibition of the substrate turnover. Preferred compounds of the invention desirably have an IC₅₀ of less than 500 nM in the factor Xa assay, preferably less than 200 nM, and more preferably less than 100 nM. Preferred compounds of the invention desirably have an IC₅₀ of less than 4.0 µM in the prothrombinase assay, preferably less than 200 nM, and more preferably less than 10 nM. Preferred compounds of the invention desirably have an IC₅₀ of greater than 1.0 µM in the thrombin assay, preferably greater than 10.0 µM, and more preferably greater than 100.0 µM.

### Amidolytic Assays for determining protease inhibition activity

Factor Xa and thrombin assays are performed at room temperature, in 0.02 M Tris HCl buffer, pH 7.5, containing 0.15 M NaCl. The rates of hydrolysis of the para-nitroanilide substrate S-2765 (Chromogenix) for factor Xa, and the substrate Chromozym TH (Boehringer Mannheim) for thrombin following preincubation of the enzyme with the test compound for 5 minutes at room temperature are determined using a Softmax 96-well plate reader (Molecular Devices), monitored at 405 nm to measure the time dependent appearance of p-nitroanilide.

The prothrombinase inhibition assay is performed in a plasma free system with modifications to the method as described by Sinha, *et al*., Thromb. Res., 75:427-436 (1994). The activity of the prothrombinase complex is determined by measuring the time course of thrombin generation using the p-nitroanilide substrate Chromozym TH. The assay consists of a 5 minute preincubation of selected compounds to be tested as inhibitors with the complex formed from factor Xa (0.5 nM), factor Va (2 nM), phosphatidyl serine:phosphatidyl choline (25:75, 20 µM) in 20 mM Tris HCl buffer, pH 7.5, containing 0.15 M NaCl, 5 mM CaCl₂ and 0.1% bovine serum albumin. Aliquots from the complex-test compound mixture are added to prothrombin (1 nM) and Chromozym TH (0.1 mM). The rate of substrate cleavage is monitored at 405 nm for two minutes. Several concentrations of a given test compound are assayed in duplicate. A standard curve of thrombin generation by an equivalent amount of untreated complex is then used for determination of percent inhibition.

The compounds of the invention exhibited inhibitory activity in the Factor Xa assay described above. The preferred compounds of the invention have IC₅₀ values less than 100 nM.

### Example 7

The antithrombotic efficacy of the compounds of this invention can readily be evaluated using a series of studies in rabbits, as described below. These studies are also useful in evaluating a compounds effects on hemostasis and its the hematological parameters.

### Antithrombotic Efficacy in a Rabbit Model of Venous Thrombosis

A rabbit deep vein thrombosis model as described by Hollenbach, *et al*., Thromb. Haemost. 71:357-362 (1994), is used to determine the *in vivo* antithrombotic activity of the compounds of the present invention. Rabbits are anesthetized with I.M. injections of Ketamine, Xylazine, and Acepromazine cocktail.

A standardized protocol consists of insertion of a thrombogenic cotton thread and copper wire apparatus into the abdominal vena cava of the anesthetized rabbit. A non-occlusive thrombus is allowed to develop in the central venous circulation and inhibition of thrombus growth is then used as a measure of the antithrombotic activity of the compound being evaluated. Test agents or control saline are administered through a marginal ear vein catheter. A femoral vein catheter is used for blood sampling prior to and during steady state infusion of the compound being evaluated. Initiation of thrombus formation will begin immediately after advancement of the cotton thread apparatus into the central venous circulation. The compounds being evaluated are administered from time=30 minutes to time=150 minutes at which point the experiment is terminated. The rabbits are euthanized and the thrombus excised by surgical dissection and characterized by weight and histology. Blood samples are then analyzed for changes in hematological and coagulation parameters.

Although the invention has been described with reference to the disclosed embodiments, those skilled in the art will readily appreciate that the specific experiments detailed are only illustrative of the invention.

## Claims

1. A compound having the formula: 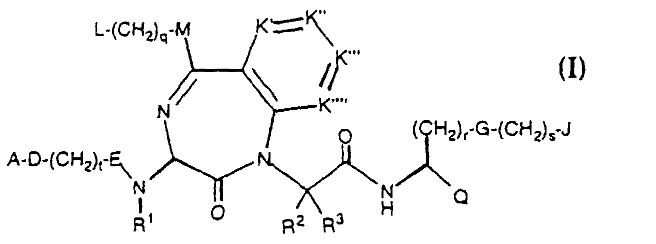 Wherein:
R¹ and R² are independently selected from the group consisting of H, C₁₋₆alkyl, C₃₋₈cycloalkyl, C₁₋₃alkylaryl, C₁₋₃alkyl-C₃₋₈cycloalkyl and aryl;
R³ is H, C₁₋₆alkyl, or R² and R³ are taken together to form a carbocyclic ring;
q is an integer from 0-1;
r is an integer from 0-4;
s is an integer from 0-1;
t is an integer from 0-4;
A is selected from the group consisting of R⁸, -NR⁸R⁹, 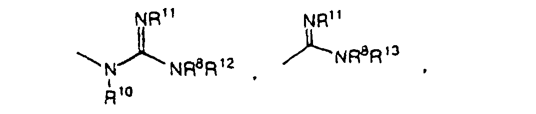 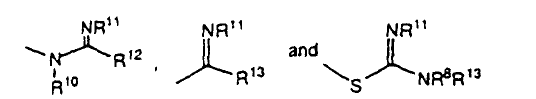 where R⁸, R⁹, R¹⁰ and R¹¹ are independently selected from the group consisting of H, -OH, C₁₋₆alkyl, aryl and C₁₋₄alkylaryl; R¹² is selected from the group consisting of H, C₁₋₆alkyl, aryl and C₁₋₄alkylaryl, or can be taken together with R¹⁰ or R¹¹ to form a 5-6 membered ring; and R¹³ is selected from the group consisting of H, C₁₋₆alkyl, aryl and C₁₋₄alkylaryl, or can be taken together with R¹¹ to form a 5-6 membered ring;
D is selected from the group consisting of a direct link, C₃₋₈cycloakyl, C₁₋₆alkenyl, C₁₋ ₆alkenylaryl, aryl and a five to ten membered heterocyclic ring system containing 1-4 heteroatoms selected from the group consisting of N, O and S;
E is selected from the group consisting of a direct link, -CO-, -SO₂-, -O-CO-, -NR¹⁴-SO₂- and -NR¹⁴-CO-, where R¹⁴ is selected from the group consisting of H, -OH, C₁₋₆alkyl, aryl and C₁₋₄alkylaryl;
G is selected from the group consisting of a direct link, C₃₋₈cycloalkyl, aryl, and a five to ten membered heterocyclic ring system containing 1-4 heteroatoms selected from the group consisting of N, O and S;
J is selected from the group consisting of R¹⁵, -NR¹⁵R¹⁶, 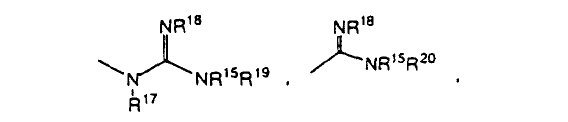 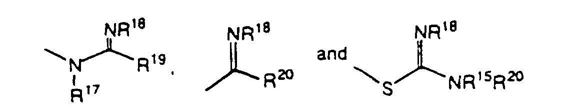 where R¹⁵, R¹⁶, R¹⁷ and R¹⁸ are independently selected from the group consisting of H, -OH, C₁₋₆alkyl, aryl and C₁₋₄alkylaryl; R¹⁹ is selected from the group consisting of H, C₁₋₆alkyl, aryl and C₁₋₄alkylaryl, or can be taken together with R¹⁷ or R¹⁸ to form a 5-6 membered ring; and R²⁰ is selected from the group consisting of H, C₁₋₆alkyl, aryl and C₁₋₄alkylaryl, or can be taken together with R¹⁸ to form a 5-6 membered ring; with the proviso that when J is R¹⁵, then G must contain at least one N atom;
K', K", K"' and K"" are independently selected from the group consisting of -CH-, -CR⁴-, -CR⁵- and -N-; with the proviso that no more than one of K', K", K'" and K"" are -CR⁴- and no more than one of K', K", K"' and K"" are -CR⁵-;
R⁴ and R⁵ are independently selected from the group consisting of H, C₁₋₆alkyl, aryl, C₁₋₆alkylaryl, C₁₋₄alkyloxy, halogen, -NO₂, -NR⁶R⁷, -NR⁶COR⁷, -OR⁶, -OCOR⁶, -COOR⁶, -CONR⁶R⁷, -CN, -CF₃, -SO₂NR⁶R⁷ and C₁₋₆alkyl-OR⁶; where R⁶ and R⁷ are independently selected from the group consisting of H, C₁₋₆alkyl, C₁₋₃alkylaryl and aryl;
L is selected from the group consisting of R³³, -NR³³R³⁴, 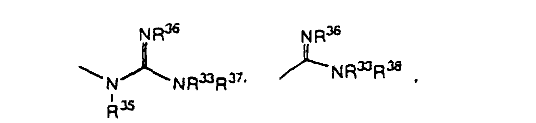 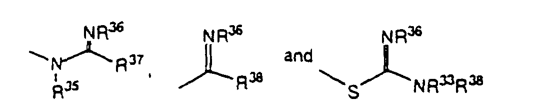 where R³³, R³⁴, R³⁵ and R³⁶ are independently selected from the group consisting of H, -OH, C₁₋₆alkyl, aryl and C₁₋₄alkylaryl; R³⁷ is selected from the group consisting of H, C₁₋₆alkyl, aryl and C₁₋₄alkylaryl, or can be taken together with R³⁵ or R³⁶ to form a 5-6 membered ring; and R³⁸ is selected from the group consisting of H, C₁₋₆alkyl, aryl and C₁₋₄alkylaryl, or can be taken together with R³⁶ to form a 5-6 membered ring;
M is selected from the group consisting of a direct link, C₁₋₆alkyl, C₃₋₈cycloalkyl, C₁₋₆alkenyl, C₁₋₆alkenylaryl, aryl, and a five to ten membered heterocyclic ring system containing 1-4 heteroatoms selected from the group consisting of N, O and S;
Q is selected from the group consisting of H, 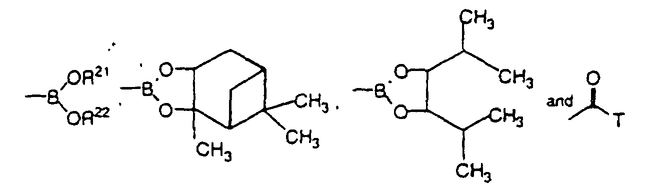 where R²¹ and R²² are independently selected from the group consisting of H, C₁₋₃alkyl and aryl; and T is selected from the group consisting of H, -COOR²³, -CONR²³R²⁴, -CF₃, -CF₂CF₃ and a group having the formula: 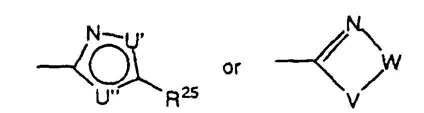 where: R²³ and R²⁴ are independently selected from the group consisting of H, C₁₋₆alkyl, aryl and C₁₋₄alkylaryl; U' and U" are independently selected from the group consisting of-O-, -S-, -N- and -NH-; with the proviso that at least one of U' or U" is -N- or -NH-; R²⁵ is selected from the group consisting of H, C₁₋₆alkyl, C₂₋₆alkenyl, C₀₋₆alkylaryl, C₂₋₆alkenylaryl, C₀₋₆alkylheterocyclo, C₂₋₆alkenylheterocyclo, -CF₃ and -CF₂CF₃; V is selected from the group consisting of -S-, -SO-, -SO₂-, -O- and -NR²⁶-, where R²⁶ is selected from the group consisting of H, C₁₋₆alkyl and benzyl; and W is selected from the group consisting of: 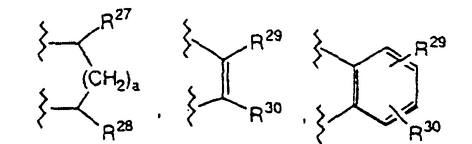 and a C₆₋₁₀ heterocyclic ring system substituted by R²⁹ and R³⁰ and containing 1-4 heteroatoms selected from N, S and O; where a: is an integer from 0-2; R²⁷ and R²⁸ are independently selected from the group consisting of H, C₁₋₆alkyl, aryl, C₁₋₆alkylaryl, -COOR³¹, -CONR³¹R³², -CN and -CF₃; and R²⁹ and R³⁰ are independently selected from the group consisting of H, C₁₋₆alkyl, aryl, C₁₋₆alkylaryl, C₁₋₄alkyloxy, halogen, -NO₂, -NR³¹R³², -NR³¹COR³², -OR^{31,} -OCOR³¹, -COOR³¹, -CONR³¹R³², -CN, -CF₃, -SO₂NR³¹R³² and C₁₋₆alkyl-OR³¹; where R³¹ and R³² are independently selected from the group consisting of H, C₁₋₆alkyl, C₁₋₃alkylaryl and aryl; and all pharmaceutically acceptable salts and optical isomers thereof.

2. The compound of Claim 1 wherein R¹ is H or C₁₋₆alkyl

3. The compound of Claim 2 wherein R¹ is H or methyl.

4. The compound of Claim 3 wherein R¹ is H.

5. The compound of Claim 1 wherein R² is H or C₁₋₆alkyl.

6. The compound of Claim 5 wherein R² is H or methyl.

7. The compound of Claim 6 wherein R² is H.

8. The compound of Claim I wherein R³ is H.

9. The compound of Claim 1 wherein R⁴ is halogen.

10. The compound of Claim 1 wherein R⁵ is halogen.

11. The compound of Claim 1 wherein the integer "r" is 3.

12. The compound of Claim I wherein the integer "s" is 0.

13. The compound of Claim 1 wherein the integer "t" is from 0-1.

14. The compound of Claim 1 wherein R⁸, R⁹, R¹⁰ and R¹¹ are independently selected from the group consisting of H and C₁₋₆alkyl.

15. The compound of Claim 1 wherein R⁸, R⁹, R¹⁰ and R¹¹ are independently selected from the group consisting of H and methyl.

16. The compound of Claim 1 wherein R¹² is H, C₁₋₆alkyl or taken together with R¹⁰ or R¹¹ to form a 5-6 membered ring.

17. The compound of Claim 16 wherein R¹² is H or methyl.

18. The compound of Claim 1 wherein R¹³ is H, C₁₋₆alkyl or taken together with R¹⁰ to form a 5-6 membered ring.

19. The compound of Claim 18 wherein R¹³ is H or methyl.

20. The compound of Claim 1 wherein D is selected from the group consisting of a direct link, C₃₋₈cycloalkyl, aryl and a five to ten membered heterocyclic ring system containing 1-4 heteroatoms selected from the group consisting of N, O and S.

21. The compound of Claim 1 wherein E is a direct link, -CO- or -SO₂-.

22. The compound of Claim 1 wherein G is a direct link.

23. The compound of Claim 1 wherein R¹⁵, R¹⁶, R¹⁷, R¹⁸, R¹⁹ and R²⁰ are independently selected from the group consisting of H and C₁₋₆alkyl.

24. The compound of Claim 23 wherein R¹⁵, R¹⁶, R¹⁷, R¹⁸, R¹⁹ and R²⁰ are independently selected from the group consisting of H and methyl.

25. The compound of Claim 1 wherein at least three of K', K", K"' and K"" are -CH-.

26. The compound of Claim 25 wherein K', K", K''' and K"" are -CH-.

27. The compound of Claim I wherein R³³, R³⁴, R³⁵ and R³⁶ are independently selected from the group consisting of H and C₁₋₆alkyl.

28. The compound of Claim 27 wherein R³³, R³⁴, R³⁵ and R³⁶ are independently selected from the group consisting of H and methyl.

29. The compound of Claim 1 wherein R³⁷ is H, C₁₋₆alkyl or taken together with R³⁵ or R³⁶ to form a 5-6 membered ring.

30. The compound of Claim 29 wherein R³⁷ is H or methyl.

31. The compound of Claim I wherein R³⁸ is H, C₁₋₆alkyl or taken together with R³⁶ to form a 5-6 membered ring.

32. The compound of Claim 31 wherein R³⁸ is H or methyl.

33. The compound of Claim 1 wherein M is a direct link, C₁₋₆alkyl, C₃₋₈cycloalkyl, aryl, or a five to ten membered heterocyclic ring system.

34. The compound of Claim 33 wherein M is C₁₋₄alkyl, aryl, or a five to ten membered heterocyclic ring system.

35. The compound of Claim 1 wherein the integer q is 0.

36. The compound of Claim 1 wherein Q is:

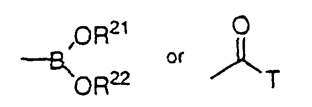

37. The compound of Claim 36 wherein R²¹ is H.

38. The compound of Claim 37 wherein R²² is H.

39. The compound of Claim 36 wherein T is H, -COOR²³, -CONR²³R²⁴ or a group having the formula:

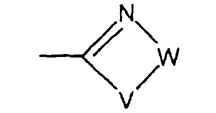

40. The compound of Claim 39 wherein R²³ is H.

41. The compound of Claim 39 wherein R²⁴ is C₁₋₄alkylaryl.

42. The compound of Claim 39 wherein V is -S-, -O- or -NR²⁶-

43. The compound of Claim 42 wherein R²⁶ is H or methyl.

44. The compound of Claim 43 wherein R²⁶ is H.

45. The compound of Claim 39 wherein W is selected from the group consisting of:

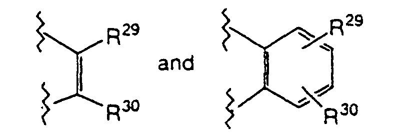

46. The compound of Claim 45 wherein W is:

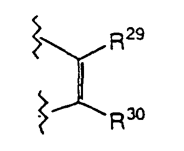

47. The compound of Claim 45 wherein R²⁹ is selected from the group consisting of H, -O-R³¹, -COOR³¹, -CONR³¹R³² or -CF₃.

48. The compound of Claim 47 wherein R²⁹ is H.

49. The compound of Claim 45 wherein R³⁰ is selected from the group consisting of H, -O-R³¹, -COOR³¹, -CONR³¹R³² or -CF₃.

50. The compound of Claim 49 wherein R³⁰ is H.

51. The compound of Claim 39 wherein W is: 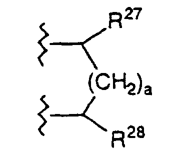 and R²⁷ is H.

52. The compound of Claim 51 wherein R²⁸ is H.

53. The compound of Claim 36 wherein T is: 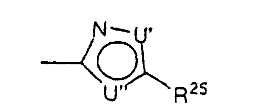 U' is O, U" is N and R²⁵ is -CF₃ or -CF₂CF₃.

54. A compound having the formula: 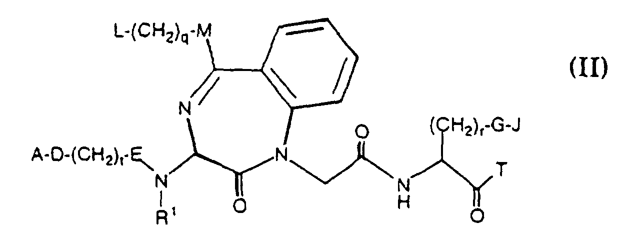 Wherein:
R¹ is selected from the group consisting of H, C₁₋₆alkyl, C₃₋₈cycloalkyl, C₁₋₃alkylaryl, C₁₋₃alkyl-C₃₋₈cycloalkyl and aryl;
q is an integer from 0-1;
r is an integer from 0-4;
t is an integer from 0-4;
A is selected from the group consisting of R⁸, -NR⁸R⁹. 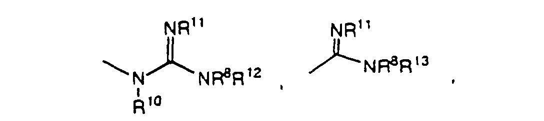 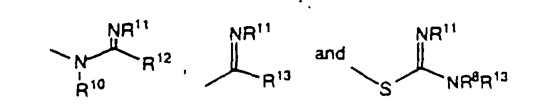 where R⁸, R⁹, R¹⁰ and R¹¹ are independently selected from the group consisting of H, -OH, C₁₋₆alkyl, aryl and C₁₋₄alkylaryl; R¹² is selected from the group consisting of H, C₁₋₆alkyl, aryl and C₁₋₄alkylaryl, or can be taken together with R¹⁰ or R¹¹ to form a 5-6 membered ring; and R¹³ is selected from the group consisting of H, C₁₋₆alkyl, aryl and C₁₋₄alkylaryl, or can be taken together with R¹¹ to form a 5-6 membered ring;
D is selected from the group consisting of a direct link, C₃₋₈cycloalkyl, C₁₋₆alkenyl, C₁₋ ₆alkenylaryl, aryl and a five to ten membered heterocyclic ring system containing 1-4 heteroatoms selected from the group consisting of N, O and S;
E is selected from the group consisting of a direct link, -CO-, -SO₂-, -O-CO-, -NR¹⁴-SO₂- and -NR¹⁴-CO-, where R¹⁴ is selected from the group consisting of H, -OH, C₁₋₆alkyl, aryl and C₁₋₄alkylaryl;
G is selected from the group consisting of a direct link, C₃₋₈cycloalkyl, aryl, and a five to ten membered heterocyclic ring system containing 1-4 heteroatoms selected from the group consisting of N, O and S;
J is selected from the group consisting of R¹⁵, -NR¹⁵R¹⁶, 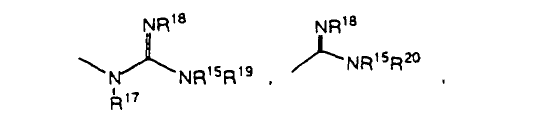 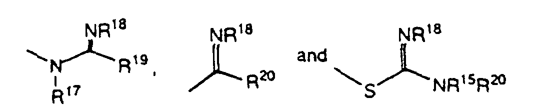 where R¹⁵, R¹⁶, R¹⁷ and R¹⁸ are independently selected from the group consisting of H, -OH, C₁₋₆alkyl, aryl and C₁₋₄alkylaryl; R¹⁹ is selected from the group consisting of H, C₁₋₆alkyl, aryl and C₁₋₄alkylaryl, or can be taken together with R¹⁷ or R¹⁸ to form a 5-6 membered ring; and R²⁰ is selected from the group consisting of H, C₁₋₆alkyl, aryl and C₁₋₄alkylaryl, or can be taken together with R¹⁸ to form a 5-6 membered ring; with the proviso that when J is R¹⁵, then G must contain at least one N atom;
L is selected from the group consisting of R³³, -NR³³R³⁴, 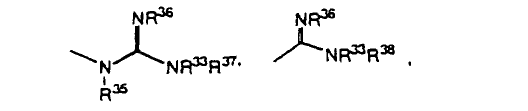 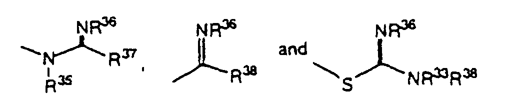 where R³³, R³⁴, R³⁵ and R³⁶ are independently selected from the group consisting of H, -OH, C₁₋₆alkyl, aryl and C₁₋₄alkylaryl; R³⁷ is selected from the group consisting of H, C₁₋₆alkyl, aryl and C₁₋₄alkylaryl, or can be taken together with R³⁵ or R³⁶ to form a 5-6 membered ring; and R³⁸ is selected from the group consisting of H, C₁₋₆alkyl, aryl and C₁₋₄alkylaryl, or can be taken together with R³⁶ to form a 5-6 membered ring;
M is selected from the group consisting of a direct link, C₁₋₆alkyl, C₃₋₈cycloalkyl. C₁₋₆alkenyl, C₁₋₆alkenylaryl, aryl, and a five to ten membered heterocyclic ring system containing 1-4 heteroatoms selected from the group consisting of N, O and S;
T is selected from the group consisting of H, -COOR²³, -CONR²³R²⁴, -CF₃, -CF₂CF₃ and a group having the formula: 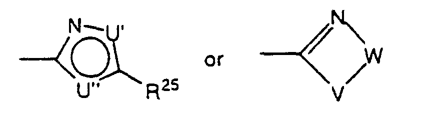 where: R²³ and R²⁴ are independently selected from the group consisting of H, C₁₋₆alkyl, aryl and C₁₋₄alkylaryl; U' and U" are independently selected from the group consisting of-O-, -S-, -N- and -NH-; with the proviso that at least one of U' or U" is -N- or -NH-; R²⁵ is selected from the group consisting of H, C₁₋₆alkyl, C₂₋₆alkenyl, C₀₋₆alkylaryl, C₂₋₆alkenylaryl, C₀₋₆alkylheterocyclo, C₂₋₆alkenylheterocyclo, -CF₃ and -CF₂CF₃; V is selected from the group consisting of -S-, -SO-, -SO₂-, -O- and -NR²⁶-, where R²⁶ is selected from the group consisting of H, C₁₋₆alkyl and benzyl; and W is selected from the group consisting of: 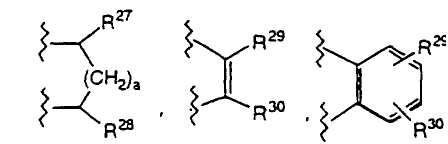 and a C₆₋₁₀ heterocyclic ring system substituted by R²⁹ and R³⁰ and containing 1-4 heteroatoms selected from N, S and O; where a: is an integer from 0-2; R²⁷ and R²⁸ are independently selected from the group consisting of H, C₁₋₆alkyl, aryl, C₁₋₆alkylaryl, -COOR³¹, -CONR³¹R³², -CN and -CF₃; and R²⁹ and R³⁰ are independently selected from the group consisting of H, C₁₋₆alkyl, aryl, C₁₋₆alkylaryl, C₁₋₄alkyloxy, halogen, -NO₂, -NR³¹R³², -NR³¹COR³², -OR³¹, -OCOR³¹, -COOR³¹, -CONR³¹R³², -CN, -CF₃, -SO₂NR³¹R³² and C₁₋₆alkyl-OR³¹; where R³¹ and R³² are independently selected from the group consisting of H, C₁₋₆alkyl, C₁₋₃alkylaryl and aryl;
and all pharmaceutically acceptable salts and optical isomers thereof.

55. A compound having the formula: 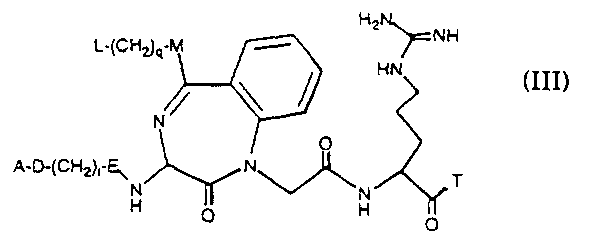 Wherein:
q is an integer from 0-1;
t is an integer from 0-4;
A is selected from the group consisting of R⁸, -NR⁸R⁹, 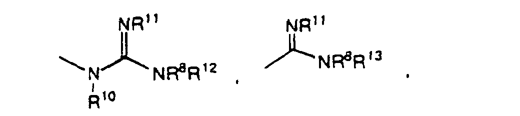 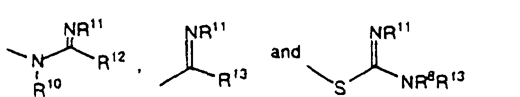 where R⁸, R⁹, R¹⁰ and R¹¹ are independently selected from the group consisting of H, -OH, C₁₋₆alkyl, aryl and C₁₋₄alkylaryl; R¹² is selected from the group consisting of H, C₁₋₆alkyl, aryl and C₁₋₄alkylaryl, or can be taken together with R¹⁰ or R¹¹ to form a 5-6 membered ring; and R¹³ is selected from the group consisting of H, C₁₋₆alkyl, aryl and C₁₋₄alkylaryl, or can be taken together with R¹¹ to form a 5-6 membered ring;
D is selected from the group consisting of a direct link, C₃₋₈cycloalkyl, C₁₋₆alkenyl, C₁₋ ₆alkenylaryl, aryl and a five to ten membered heterocyclic ring system containing 1-4 heteroatoms selected from the group consisting of N, O and S;
E is selected from the group consisting of a direct link, -CO-, -SO₂-, -O-CO-, -NR¹⁴-SO₂- and -NR¹⁴-CO-, where R¹⁴ is selected from the group consisting of H, -OH, C₁₋₆alkyl, aryl and C₁₋₄alkylaryl;
L is selected from the group consisting of R³³, -NR³³R³⁴, 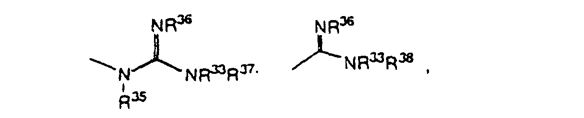 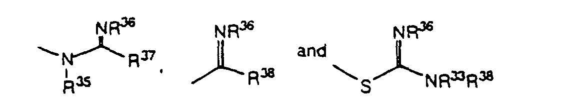 where R³³, R³⁴, R³⁵ and R³⁶ are independently selected from the group consisting of H, -OH, C₁₋₆alkyl, aryl and C₁₋₄alkylaryl; R³⁷ is selected from the group consisting of H, C₁₋₆alkyl, aryl and C₁₋₄alkylaryl, or can be taken together with R³⁵ or R³⁶ to form a 5-6 membered ring; and R³⁸ is selected from the group consisting of H, C₁₋₆alkyl, aryl and C₁₋₄alkylaryl, or can be taken together with R³⁶ to form a 5-6 membered ring;
M is selected from the group consisting of a direct link, C₁₋₆alkyl, C₃₋₈cycloalkyl, C₁₋₆alkenyl, C₁₋₆alkenylaryl, aryl, and a five to ten membered heterocyclic ring system containing 1-4 heteroatoms selected from the group consisting of N, O and S;
T is selected from the group consisting of H, -COOR²³, -CONR²³R²⁴, -CF₃, -CF₂CF₃ and a group having the formula: 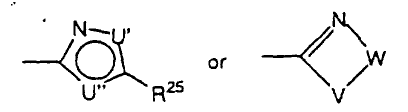 where: R²³ and R²⁴ are independently selected from the group consisting of H, C₁₋₆alkyl, aryl and C₁₋₄alkylaryl; U' and U" are independently selected from the group consisting of-O-, -S-, -N- and -NH-; with the proviso that at least one of U' or U" is -N- or -NH-; R²⁵ is selected from the group consisting of H, C₁₋₆alkyl, C₂₋₆alkenyl, C₀₋₆alkylaryl, C₂₋₆alkenylaryl, C₀₋₆alkylheterocyclo, C₂₋₆alkenylheterocyclo, -CF₃ and -CF₂CF₃; V is selected from the group consisting of -S-, -SO-, -SO₂-, -O- and -NR²⁶-, where R²⁶ is selected from the group consisting of H, C₁₋₆alkyl and benzyl; and W is selected from the group consisting of: 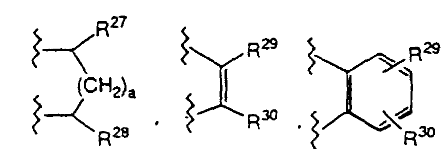 and a C₆₋₁₀ heterocyclic ring system substituted by R²⁹ and R³⁰ and containing 1-4 heteroatoms selected from N, S and O ; where : a is an integer from 0-2 ; R²⁷ and R²⁸ are independently selected from the group consisting of H, C₁₋₆alkyl, aryl, C₁₋₆alkylaryl, -COOR³¹, -CONR³¹R³², -CN and -CF₃ ; and R²⁹ and R³⁰ are independently selected from the group consisting of H, C₁₋₆alkyl, aryl, C₁₋₆alkylaryl, C₁₋₄alkyloxy, halogen, -NO₂, -NR³¹R³², -NR³¹COR³², -OR³¹, -OCOR³¹, -COOR³¹, -CONR³¹R³², -CN, -CF₃, -SO₂NR³¹R³² and C₁₋₆alkyl-OR³¹ ; where R³¹ and R³² are independently selected from the group consisting of H, C₁₋₆alkyl, C₁₋₃ alkylaryl and aryl ;
and all pharmaceutically acceptable salts and optical isomers thereof.

56. A pharmaceutical composition for preventing or treating a condition in a mammal **characterized by** undesired thrombosis comprising a pharmaceutically acceptable carrier and the compound of Claim 1.

57. The use of a therapeutically effective amount of the compound of Claim 1 for the manufacture of a drug for preventing or treating a condition in a mammal **characterized by** undesired thrombosis.

58. The use of Claim 57, wherein the condition is selected from the group consisting of: the treatment or prevention of unstable angina, refractory angina, myocardial infarction, transient ischemic attacks, thrombotic stroke, embolic stroke, disseminated intravascular coagulation including the treatment of septic shock, deep venous thrombosis in the prevention of pulmonary embolism or the treatment of reocclusion or restenosis of reperfused coronary arteries, deep venous thrombosis, pulmonary embolism, myocardial infarction, stroke, thromboembolic complications of surgery and peripheral arterial occlusion, occlusive coronary thrombus formation resulting from either thrombolytic therapy or percutaneous transluminal coronary angioplasty, thrombus formation in the venous vasculature and disseminated intravascular coagulopathy.

59. A method for inhibiting the coagulation of biological samples, comprising the administration of the compound of Claim 1.

## Patentansprüche

1. Verbindung mit der Formel: 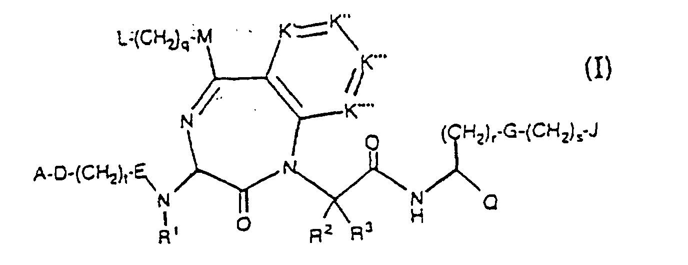 wobei:
R¹ und R² unabhängig ausgewählt sind aus der Gruppe, bestehend aus H, C₁₋₆-Alkyl, C₃₋₈-cycloalkyl, C₁₋₃-Alkylaryl, C₁₋₃-Alkyl-C₃₋₈-cycloalkyl und Aryl;
R³ gleich H, C₁₋₆-Alkyl ist oder R² und R³ zusammen einen carbocyclischen Ring ausbilden;
q eine ganze Zahl von 0-1 ist;
r eine ganze Zahl von 0-4 ist;
s eine ganze Zahl von 0-1 ist;
t eine ganze Zahl von 0-4 ist;
A ausgewählt ist aus der Gruppe, bestehend aus R⁸, -NR⁸R⁹, 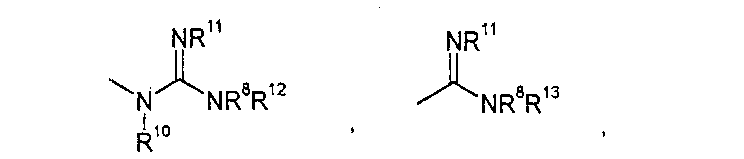 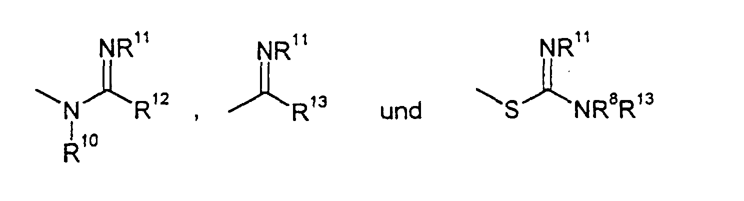 wobei R⁸, R⁹, R¹⁰ und R¹¹ unabhängig ausgewählt sind aus der Gruppe, bestehend aus H, -OH, C₁₋₆-Alkyl, Aryl und C₁₋₄-Alkylaryl; R¹² ausgewählt ist aus der Gruppe, bestehend aus H, C₁₋₆-Alkyl, Aryl und C₁₋₄-Alkylaryl, oder zusammen mit R¹⁰ oder R¹¹ einen 5-6-gliedrigen Ring ausbilden kann; und R¹³ ausgewählt ist aus der Gruppe, bestehend aus H, C₁₋₆-Alkyl, Aryl und C₁₋₄-Alkylaryl, oder zusammen mit R¹¹ einen 5-6-gliedrigen Ring ausbilden kann;
D ausgewählt ist aus der Gruppe, bestehend aus einer direkten Bindung, C₃₋₈-Cycloalkyl, C₁₋₆-Alkenyl, C₁₋₆-Alkenylaryl, Aryl und einem fünf- bis zehngliedrigen heterocyclischen Ringsystem, welches 1-4 Heteroatome enthält, die aus der aus N, O und S bestehenden Gruppe ausgewählt sind;
E ausgewählt ist aus der Gruppe, bestehend aus einer direkten Bindung, -CO-, -SO₂-, -O-CO-, -NR¹⁴-SO₂- und -NR¹⁴-CO-, wobei R¹⁴ ausgewählt ist aus der Gruppe, bestehend aus H, -OH, C₁₋₆-Alkyl, Aryl und C₁₋₄-Alkylaryl;
G ausgewählt ist aus der Gruppe, bestehend aus einer direkten Bindung, C₃₋₈-Cycloalkyl, Aryl und einem fünf- bis zehngliedrigen heterocyclischen Ringsystem, welches 1-4 Heteroatome enthält, die aus der aus N, O und S bestehenden Gruppe ausgewählt sind;
J ausgewählt ist aus der Gruppe, bestehend aus R¹⁵, -NR¹⁵R¹⁶, 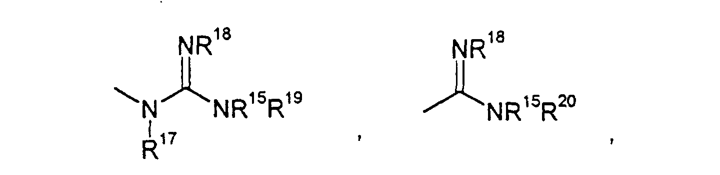 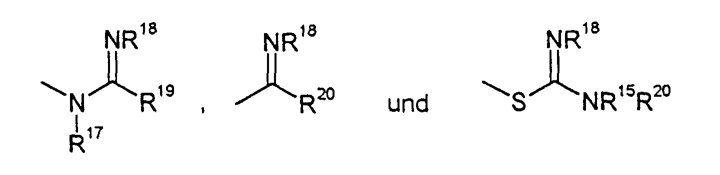 wobei R¹⁵, R¹⁶, R¹⁷ und R¹⁸ unabhängig ausgewählt sind aus der Gruppe, bestehend aus H, -OH, C₁₋₆-Alkyl, Aryl und C₁₋₄-Alkylaryl; R¹⁹ ausgewählt ist aus der Gruppe, bestehend aus H, C₁₋₆-Alkyl, Aryl und C₁₋₄-Alkylaryl oder zusammen mit R¹⁷ oder R¹⁸ einen 5-6-gliedrigen Ring ausbilden kann; und R²⁰ ausgewählt ist aus der Gruppe, bestehend aus H, C₁₋₆-Alkyl, Aryl und C₁₋₄-Alkylaryl oder zusammen mit R¹⁸ einen 5-6-gliedrigen Ring ausbilden kann; unter der Bedingung, daß wenn J gleich R¹⁵ ist, G dann mindestens ein N-Atom enthalten muß;
K', K", K"' und K"" unabhängig ausgewählt sind aus der Gruppe, bestehend aus -CH-, -CR⁴-, -CR⁵- und -N-; unter der Bedingung, daß nicht mehr als eines von K', K", K"' und K"" gleich -CR⁴- ist und nicht mehr als eines von K', K", K"' und K"" gleich -CR⁵- ist;
R⁴ und R⁵ unabhängig ausgewählt sind aus der Gruppe, bestehend aus H, C₁₋₆-Alkyl, Aryl, C₁₋₆-Alkylaryl, C₁₋₄-Alkyloxy, Halogen, -NO₂, -NR⁶R⁷, -NR⁶COR⁷, -OR⁶, -OCOR⁶, -COOR⁶, -CONR⁶R⁷, -CN, -CF₃, -SO₂NR⁶R⁷ und C₁₋₆-Alkyl-OR⁶; wobei R⁶ und R⁷ unabhängig ausgewählt sind aus der Gruppe, bestehend aus H, C₁₋₆-Alkyl, C₁₋₃-Alkylaryl und Aryl;
L ausgewählt ist aus der Gruppe, bestehend aus R³³, -NR³³R³⁴, 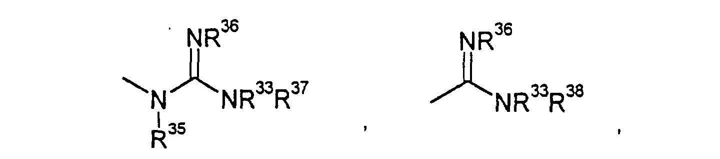 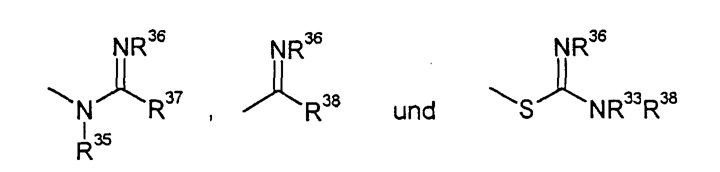 wobei R³³, R³⁴, R³⁵ und R³⁶ unabhängig ausgewählt sind aus der Gruppe, bestehend aus H, -OH, C₁₋₆-Alkyl, Aryl und C₁₋₄-Alkylaryl; R³⁷ ausgewählt ist aus der Gruppe, bestehend aus H, C₁₋₆-Alkyl, Aryl und C₁₋₄-Alkylaryl oder zusammen mit R³⁵ oder R³⁶ einen 5-6-gliedrigen Ring ausbilden kann; und R³⁸ ausgewählt ist aus der Gruppe, bestehend aus H, C₁₋₆-Alkyl, Aryl und C₁₋₄-Alkylaryl, oder zusammen mit R³⁶ einen 5-6-gliedrigen Ring ausbilden kann;
M ausgewählt ist aus der Gruppe, bestehend aus einer direkten Bindung, C₁₋₆-Alkyl, C₃₋₈-Cycloalkyl, C₁₋₆-Alkenyl, C₁₋₆-Alkenylaryl, Aryl und einem fünfbis zehngliedrigen heterocyclischen Ringsystem, welches 1-4 Heteroatome enthält, die aus der aus N, O und S bestehenden Gruppe ausgewählt sind;
Q ausgewählt ist aus der Gruppe, bestehend aus H, 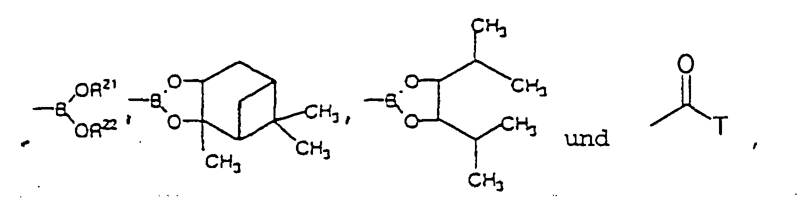 wobei R²¹ und R²² unabhängig ausgewählt sind aus der Gruppe, bestehend aus H, C₁₋₃-Alkyl und Aryl; und T ausgewählt ist aus der Gruppe, bestehend aus H, -COOR²³, -CONR²³R²⁴, -CF₃, -CF₂CF₃ und einer Gruppe mit der Formel: 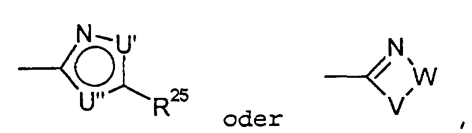 wobei R²³ und R²⁴ unabhängig ausgewählt sind aus der Gruppe, bestehend aus H, C₁₋₆-Alkyl, Aryl und C₁₋₄-Alkylaryl; U' und U" unabhängig ausgewählt sind aus der Gruppe, bestehend aus -O-, -S-, -N- und -NH-; unter der Bedingung, daß mindestens eines von U' oder U" gleich -N- oder -NH- ist; R²⁵ ausgewählt ist aus der Gruppe, bestehend aus H, C₁₋₆-Alkyl, C₂₋₆-Alkenyl, C₀₋₆-Alkylaryl, C₂₋₆-Alkenylaryl, C₀₋₆-Alkylheterocyclo, C₂₋₆-Alkenylheterocyclo, -CF₃ und -CF₂CF₃; V ausgewählt ist aus der Gruppe, bestehend aus -S-, -SO-, -SO₂-, -O- und -NR²⁶-, wobei R²⁶ ausgewählt ist aus der Gruppe, bestehend aus H, C₁₋₆-Alkyl und Benzyl; und W ausgewählt ist aus der Gruppe, bestehend aus: 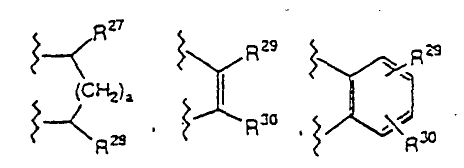 und einem heterocyclischen C₆₋₁₀-Ringsystem, welches substituiert ist mit R²⁹ und R³⁰ und 1-4 Heteroatome enthält, die ausgewählt sind aus N, S und O; wobei a eine ganze Zahl von 0-2 ist; R²⁷ und R²⁸ unabhängig ausgewählt sind aus der Gruppe, bestehend aus H, C₁₋₆-Alkyl, Aryl, C₁₋₆-Alkylaryl, -COOR³¹, -CONR³¹R³², -CN und -CF₃; und R²⁹ und R³⁰ unabhängig ausgewählt sind aus der Gruppe, bestehend aus H, C₁₋₆-Alkyl, Aryl, C₁₋₆-Alkylaryl, C₁₋₄-Alkyloxy, Halogen, -NO₂, -NR³¹R³², -NR³¹COR³², -OR³¹, -OCOR³¹,-COOR³¹, -CONR³¹R³², -CN, -CF₃, -SO₂NR³¹R³² und C₁₋₆-Alkyl-OR³¹; wobei R³¹ und R³² unabhängig ausgewählt sind aus der Gruppe, bestehend aus H, C₁₋₆-Alkyl, C₁₋₃-Alkylaryl und Aryl;
und alle pharmazeutisch akzeptablen Salze und optischen Isomeren davon.

2. Verbindung nach Anspruch 1 wobei R¹ gleich H oder C₁₋₆-Alkyl ist.

3. Verbindung nach Anspruch 2 wobei R¹ gleich H oder Methyl ist.

4. Verbindung nach Anspruch 3 wobei R¹ gleich H ist.

5. Verbindung nach Anspruch 1 wobei R² gleich ⁻H oder C₁₋₆-Alkyl ist.

6. Verbindung nach Anspruch 5 wobei R² gleich H oder Methyl ist.

7. Verbindung nach Anspruch 6 wobei R² gleich H ist.

8. Verbindung nach Anspruch 1 wobei R³ gleich H ist.

9. Verbindung nach Anspruch 1 wobei R⁴ gleich Halogen ist.

10. Verbindung nach Anspruch 1 wobei R⁵ gleich Halogen ist.

11. Verbindung nach Anspruch 1 wobei die ganze Zahl "r" gleich 3 ist.

12. Verbindung nach Anspruch 1 wobei die ganze Zahl "s" gleich 0 ist.

13. Verbindung nach Anspruch 1 wobei die ganze Zahl "t" gleich 0-1 ist.

14. Verbindung nach Anspruch 1 wobei R⁸, R⁹, R¹⁰ und R¹¹ unabhängig ausgewählt sind aus der Gruppe, bestehend aus H und C₁₋₆-Alkyl.

15. Verbindung nach Anspruch 1 wobei R⁸, R⁹, R¹⁰ und R¹¹ unabhängig ausgewählt sind aus der Gruppe, bestehend aus H und Methyl.

16. Verbindung nach Anspruch 1 wobei R¹² gleich H ist, C₁₋₆-Alkyl ist oder zusammen mit R¹⁰ oder R¹¹ einen 5-6-gliedrigen Ring ausbildet.

17. Verbindung nach Anspruch 16 wobei R¹² gleich H oder Methyl ist.

18. Verbindung nach Anspruch 1 wobei R¹³ gleich H ist, C₁₋₆-Alkyl ist oder zusammen mit R¹⁰ einen 5-6-gliedrigen Ring ausbildet.

19. Verbindung nach Anspruch 18 wobei R¹³ gleich H oder Methyl ist.

20. Verbindung nach Anspruch 1 wobei D ausgewählt ist aus der Gruppe, bestehend aus einer direkten Bindung, C₃₋₈-Cycloalkyl, Aryl und einem fünf- bis zehngliedrigen heterocyclischen Ringsystem, welches 1-4 Heteroatome enthält, die aus der aus N, O und S bestehenden Gruppe ausgewählt sind.

21. Verbindung nach Anspruch 1 wobei E eine direkte Bindung, -CO- oder -SO₂- ist.

22. Verbindung nach Anspruch 1 wobei G eine direkte Bindung ist.

23. Verbindung nach Anspruch 1 wobei R¹⁵, R¹⁶, R¹⁷, R¹⁸, R¹⁹ und R²⁰ unabhängig ausgewählt sind aus der Gruppe, bestehend aus H und C₁₋₆-Alkyl.

24. Verbindung nach Anspruch 23 wobei R¹⁵ R¹⁶ R¹⁷ R¹⁸ R¹⁹ und R²⁰ unabhängig ausgewählt sind aus der Gruppe, bestehend aus H und Methyl.

25. Verbindung nach Anspruch 1 wobei mindestens drei von K', K", K"' und K"" gleich -CH- sind.

26. Verbindung nach Anspruch 25 wobei K', K", K"' und K"" gleich -CH- sind.

27. Verbindung nach Anspruch 1 wobei R³³, R³⁴, R³⁵ und R³⁶ unabhängig ausgewählt sind aus der Gruppe, bestehend aus H und C₁₋₆-Alkyl.

28. Verbindung nach Anspruch 27 wobei R³³, R³⁴, R³⁵ und R³⁶ unabhängig ausgewählt sind aus der Gruppe, bestehend aus H und Methyl.

29. Verbindung nach Anspruch 1 wobei R³⁷ gleich H ist, C₁₋₆-Alkyl ist oder zusammen mit R³⁵ oder R³⁶ einen 5-6-gliedrigen Ring ausbildet.

30. Verbindung nach Anspruch 29 wobei R³⁷ gleich H oder Methyl ist.

31. Verbindung nach Anspruch 1 wobei R³⁸ gleich H ist, C₁₋₆-Alkyl ist oder zusammen mit R³⁶ einen 5-6-gliedrigen Ring ausbildet.

32. Verbindung nach Anspruch 31 wobei R³⁸ gleich H oder Methyl ist.

33. Verbindung nach Anspruch 1 wobei M eine direkte Bindung, C₁₋₆-Alkyl, C₃₋₈-Cycloalkyl, Aryl oder ein fünf- bis zehngliedriges heterocyclisches Ringsystem ist.

34. Verbindung nach Anspruch 33 wobei M gleich C₁₋₄-Alkyl, Aryl oder ein fünf- bis zehngliedriges heterocyclisches Ringsystem ist.

35. Verbindung nach Anspruch 1 wobei die ganze Zahl q gleich 0 ist.

36. Verbindung nach Anspruch 1 wobei Q gleich 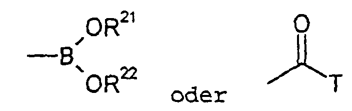 ist.

37. Verbindung nach Anspruch 36 wobei R²¹ gleich H ist.

38. Verbindung nach Anspruch 37 wobei R²² gleich H ist.

39. Verbindung nach Anspruch 36 wobei T gleich H, -COOR²³, -CONR²³R²⁴ oder eine Gruppe der Formel 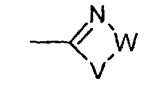 ist.

40. Verbindung nach Anspruch 39 wobei R²³ gleich H ist.

41. Verbindung nach Anspruch 39 wobei R²⁴ gleich C₁₋₄-Alkylaryl ist.

42. Verbindung nach Anspruch 39 wobei V gleich -S-, -O- oder -NR²⁶- ist.

43. Verbindung nach Anspruch 42 wobei R²⁶ gleich H oder Methyl ist.

44. Verbindung nach Anspruch 43 wobei R²⁶ gleich H ist.

45. Verbindung nach Anspruch 39 wobei W ausgewählt ist aus der Gruppe, bestehend aus

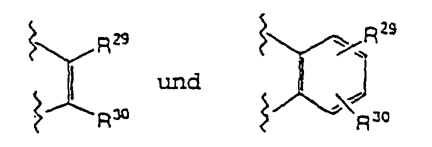

46. Verbindung nach Anspruch 45 wobei W gleich 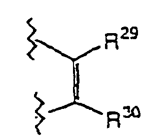 ist

47. Verbindung nach Anspruch 45 wobei R²⁹ ausgewählt ist aus der Gruppe, bestehend aus H, -O-R³¹, -COOR³¹, -CONR³¹R³² or -CF₃.

48. Verbindung nach Anspruch 47 wobei R²⁹ gleich H ist.

49. Verbindung nach Anspruch 45 wobei R³⁰ ausgewählt ist aus der Gruppe, bestehend aus H, -O-R³¹, -COOR³¹, -CONR³¹R³² oder -CF₃.

50. Verbindung nach Anspruch 49 wobei R³⁰ gleich H ist.

51. Verbindung nach Anspruch 39 wobei W gleich 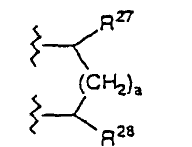 ist und R²⁷ gleich H ist.

52. Verbindung nach Anspruch 51 wobei R²⁸ gleich H ist.

53. Verbindung nach Anspruch 36 wobei T gleich 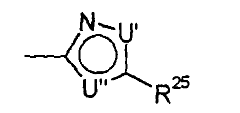 ist, U' gleich O ist, U" gleich N ist und R²⁵ gleich -CF₃ oder -CF₂CF₃ ist.

54. Verbindung mit der Formel: 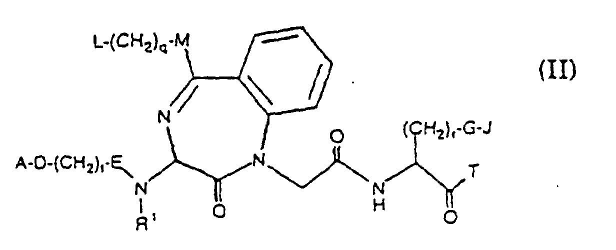 wobei:
R¹ aus der Gruppe ausgewählt ist, bestehend aus H, C₁₋₆-Alkyl, C₃₋₈-Cycloalkyl, C₁₋₃-Alkylaryl, C₁₋₃-Alkyl-C₃₋₈-cycloalkyl und Aryl;
q eine ganze Zahl von 0-1 ist;
r eine ganze Zahl von 0-4 ist;
t eine ganze Zahl von 0-4 ist;
A ausgewählt ist aus der Gruppe, bestehend aus R⁸, -NR⁸R⁹, 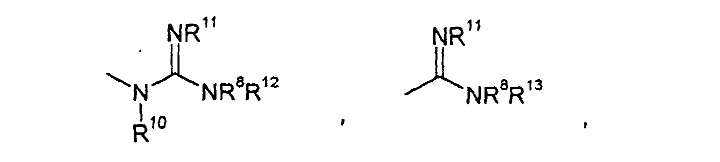 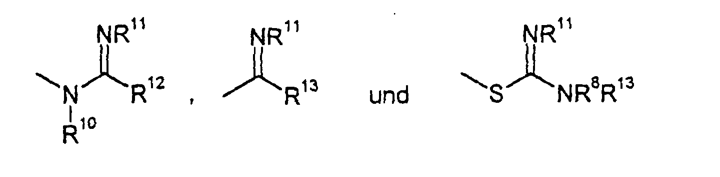 wobei R⁸, R⁹, R¹⁰ und R¹¹ unabhängig ausgewählt sind aus der Gruppe, bestehend aus H, -OH, C₁₋₆-Alkyl, Aryl und C₁₋₄-Alkylaryl; R¹² ausgewählt ist aus der Gruppe, bestehend aus H, C₁₋₆-Alkyl, Aryl und C₁₋₄-Alkylaryl, oder zusammen mit R¹⁰ oder R¹¹ einen 5-6-gliedrigen Ring ausbilden kann; und R¹³ ausgewählt ist aus der Gruppe, bestehend aus H, C₁₋₆-Alkyl, Aryl und C₁₋₄-Alkylaryl, oder zusammen mit R¹¹ einen 5-6-gliedrigen Ring ausbilden kann;
D ausgewählt ist aus der Gruppe, bestehend aus einer direkten Bindung, C₃₋₈-Cycloalkyl, C₁₋₆-Alkenyl, C₁₋₆-Alkenylaryl, Aryl und einem fünf- bis zehngliedrigen heterocyclischen Ringsystem, welches 1-4 Heteroatome enthält, die aus der aus N, O und S bestehenden Gruppe ausgewählt sind;
E ausgewählt ist aus der Gruppe, bestehend aus einer direkten Bindung, -CO-, -SO₂-, -O-CO-, -NR¹⁴-SO₂- und -NR¹⁴-CO-, wobei R¹⁴ ausgewählt ist aus der Gruppe, bestehend aus H, -OH, C₁₋₆-Alkyl, Aryl und C₁₋₄-Alkylaryl;
G ausgewählt ist aus der Gruppe, bestehend aus einer direkten Bindung, C₃₋₈-Cycloalkyl, Aryl und einem fünf- bis zehngliedrigen heterocyclischen Ringsystem, welches 1-4 Heteroatome enthält, die aus der aus N, O und S bestehenden Gruppe ausgewählt sind;
J ausgewählt ist aus der Gruppe, bestehend aus R¹⁵, -NR¹⁵R¹⁶, 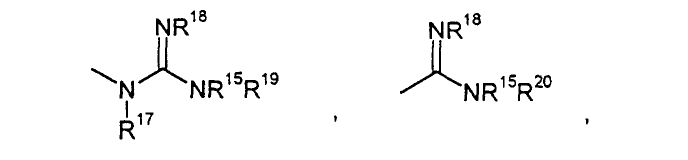 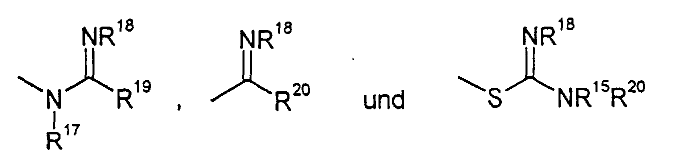 wobei R¹⁵, R¹⁶, R¹⁷ und R¹⁸ unabhängig ausgewählt sind aus der Gruppe, bestehend aus H, -OH, C₁₋₆-Alkyl, Aryl und C₁₋₄-Alkylaryl; R¹⁹ ausgewählt ist aus der Gruppe, bestehend aus H, C₁₋₆-Alkyl, Aryl und C₁₋₄-Alkylaryl oder zusammen mit R¹⁷ oder R¹⁸ einen 5-6-gliedrigen Ring ausbilden kann; und R²⁰ ausgewählt ist aus der Gruppe, bestehend aus H, C₁₋₆-Alkyl, Aryl und C₁₋₄-Alkylaryl oder zusammen mit R¹⁸ einen 5-6-gliedrigen Ring ausbilden kann; unter der Bedingung, daß wenn J gleich R¹⁵ ist, G dann mindestens ein N-Atom enthalten muß;
L ausgewählt ist aus der Gruppe, bestehend aus R³³, -NR³³R³⁴, 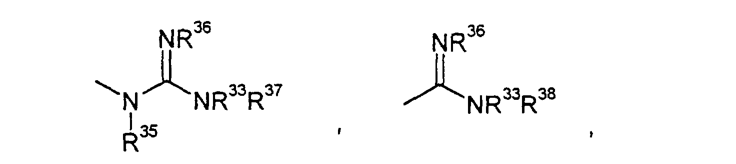 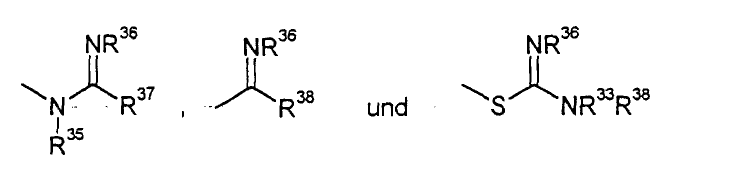 wobei R³³, R³⁴, R³⁵ und R³⁶ unabhängig ausgewählt sind aus der Gruppe, bestehend aus H, -OH, C₁₋₆-Alkyl, Aryl und C₁₋₄-Alkylaryl; R³⁷ ausgewählt ist aus der Gruppe, bestehend aus H, C₁₋₆-Alkyl, Aryl und C₁₋₄-Alkylaryl oder zusammen mit R³⁵ oder R³⁶ einen 5-6-gliedrigen Ring ausbilden kann; und R³⁸ ausgewählt ist aus der Gruppe, bestehend aus H, C₁₋₆-Alkyl, Aryl und C₁₋₄-Alkylaryl, oder zusammen mit R³⁶ einen 5-6-gliedrigen Ring ausbilden kann;
M ausgewählt ist aus der Gruppe, bestehend aus einer direkten Bindung, C₁₋₆-Alkyl, C₃₋₈-Cycloalkyl, C₁₋₆-Alkenyl, C₁₋₆-Alkenylaryl, Aryl und einem fünfbis zehngliedrigen heterocyclischen Ringsystem, welches 1-4 Heteroatome enthält, die aus der aus N, O und S bestehenden Gruppe ausgewählt sind;
T ausgewählt ist aus der Gruppe, bestehend aus H, -COOR²³, -CONR²³R²⁴, -CF₃, -CF₂CF₃ und einer Gruppe mit der Formel: 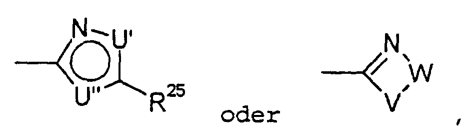 wobei R²³ und R²⁴ unabhängig ausgewählt sind aus der Gruppe, bestehend aus H, C₁₋₆-Alkyl, Aryl und C₁₋₄-Alkylaryl; U' und U" unabhängig ausgewählt sind aus der Gruppe, bestehend aus -O-, -S-, -N- und -NH-; unter der Bedingung, daß mindestens eines von U' oder U" gleich -N- oder -NH- ist; R²⁵ ausgewählt ist aus der Gruppe, bestehend aus H, C₁₋₆-Alkyl, C₂₋₆-Alkenyl, C₀₋₆-Alkylaryl, C₂₋₆-Alkenylaryl, C₀₋₆-Alkylheterocyclo, C₂₋₆-Alkenylheterocyclo, -CF3 und -CF₂CF₃; V ausgewählt ist aus der Gruppe, bestehend aus -S-, -SO-, -SO₂-, -O- und -NR²⁶-, wobei R²⁶ ausgewählt ist aus der Gruppe, bestehend aus H, C₁₋₆-Alkyl und Benzyl; und W ausgewählt ist aus der Gruppe, bestehend aus: 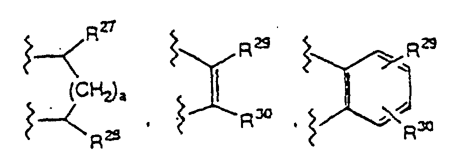 und einem heterocyclischen C₆₋₁₀-Ringsystem, welches substituiert ist mit R²⁹ und R³⁰ und 1-4 Heteroatome enthält, die ausgewählt sind aus N, S und O; wobei a eine ganze Zahl von 0-2 ist; R²⁷ und R²⁸ unabhängig ausgewählt sind aus der Gruppe, bestehend aus H, C₁₋₆-Alkyl, Aryl, C₁₋₆-Alkylaryl, -COOR³¹, -CONR³¹R³², -CN und -CF₃; und R²⁹ und R³⁰ unabhängig ausgewählt sind aus der Gruppe, bestehend aus H, C₁₋₆-Alkyl, Aryl, C₁₋₆-Alkylaryl, C₁₋₄-Alkyloxy, Halogen, -NO₂, -NR³³R³², -NR³¹COR³², -OR³¹, -OCOR³¹,-COOR³¹, -CONR³¹R³², -CN, -CF₃, -SO₂NR³¹R³² und C₁₋₆-Alkyl-OR³¹; wobei R³¹ und R³² unabhängig ausgewählt sind aus der Gruppe, bestehend aus H, C₁₋₆-Alkyl, C₁₋₃-Alkylaryl und Aryl;
und alle pharmazeutisch akzeptablen Salze und optischen Isomeren davon.

55. Verbindung mit der Formel: 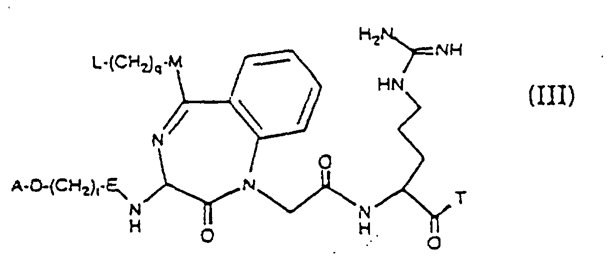 wobei:
q eine ganze Zahl von 0-1 ist;
t eine ganze Zahl von 0-4 ist;
A ausgewählt ist aus der Gruppe, bestehend aus R⁸, -NR⁸R⁹, 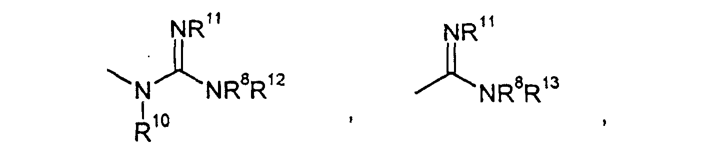 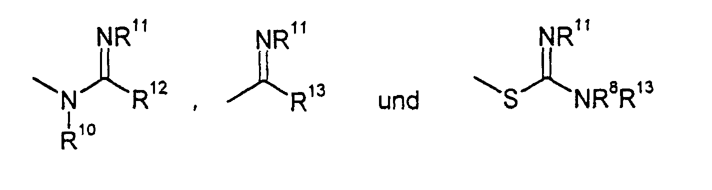 wobei R⁸, R⁹, R¹⁰ und R¹¹ unabhängig ausgewählt sind aus der Gruppe, bestehend aus H, -OH, C₁₋₆-Alkyl, Aryl und C₁₋₄-Alkylaryl; R¹² ausgewählt ist aus der Gruppe, bestehend aus H, C₁₋₆-Alkyl, Aryl und C₁₋₄-Alkylaryl, oder zusammen mit R¹⁰ oder R¹¹ einen 5-6-gliedrigen Ring ausbilden kann; und R¹³ ausgewählt ist aus der Gruppe, bestehend aus H, C₁₋₆-Alkyl, Aryl und C₁₋₄-Alkylaryl, oder zusammen mit R¹¹ einen 5-6-gliedrigen Ring ausbilden kann;
D ausgewählt ist aus der Gruppe, bestehend aus einer direkten Bindung, C₃₋₈-Cycloalkyl, C₁₋₆-Alkenyl, C₁₋₆-Alkenylaryl, Aryl und einem fünf- bis zehngliedrigen heterocyclischen Ringsystem, welches 1-4 Heteroatome enthält, die aus der aus N, O und S bestehenden Gruppe ausgewählt sind;
E ausgewählt ist aus der Gruppe, bestehend aus einer direkten Bindung, -CO-, -SO₂-, -O-CO-, -NR¹⁴-SO₂- und -NR¹⁴-CO-, wobei R¹⁴ ausgewählt ist aus der Gruppe, bestehend aus H, -OH, C₁₋₆-Alkyl, Aryl und C₁₋₄-Alkylaryl;
L ausgewählt ist aus der Gruppe, bestehend aus R³³, -NR³³R³⁴, 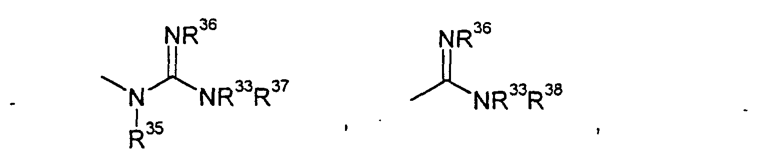 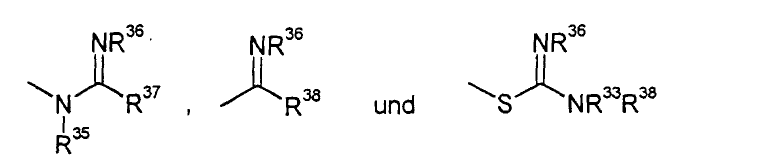 wobei R³³, R³⁴, R³⁵ und R³⁶ unabhängig ausgewählt sind aus der Gruppe, bestehend aus H, -OH, C₁₋₆-Alkyl, Aryl und C₁₋₄-Alkylaryl; R³⁷ ausgewählt ist aus der Gruppe, bestehend aus H, C₁₋₆-Alkyl, Aryl und C₁₋₄-Alkylaryl oder zusammen mit R³⁵ oder R³⁶ einen 5-6-gliedrigen Ring ausbilden kann; und R³⁸ ausgewählt ist aus der Gruppe, bestehend aus H, C₁₋₆-Alkyl, Aryl und C₁₋₄-Alkylaryl, oder zusammen mit R³⁶ einen 5-6-gliedrigen Ring ausbilden kann;
M ausgewählt ist aus der Gruppe, bestehend aus einer direkten Bindung, C₁₋₆-Alkyl, C₃₋₈-Cycloalkyl, C₁₋₆-Alkenyl, C₁₋₆-Alkenylaryl, Aryl und einem fünfbis zehngliedrigen heterocyclischen Ringsystem, welches 1-4 Heteroatome enthält, die aus der aus N, O und S bestehenden Gruppe ausgewählt sind;
T ausgewählt ist aus der Gruppe, bestehend aus H, -COOR²³, -CONR²³R²⁴, -CF₃, -CF₂CF₃ und einer Gruppe mit der Formel: 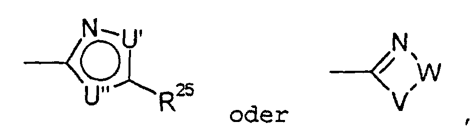 wobei R²³ und R²⁴ unabhängig ausgewählt sind aus der Gruppe, bestehend aus H, C₁₋₆-Alkyl, Aryl und C₁₋₄-Alkylaryl; U' und U" unabhängig ausgewählt sind aus der Gruppe, bestehend aus -O-, -S-, -N- und -NH-; unter der Bedingung, daß mindestens eines von U' oder U" gleich -N- oder -NH- ist; R²⁵ ausgewählt ist aus der Gruppe, bestehend aus H, C₁₋₆-Alkyl, C₂₋₆-Alkenyl, C₀₋₆-Alkylaryl, C₂₋₆-Alkenylaryl, C₀₋₆-Alkylheterocyclo, C₂₋₆-Alkenylheterocyclo, -CF₃ und -CF₂CF₃; V ausgewählt ist aus der Gruppe, bestehend aus -S-, -SO-, -SO₂-, -O- und -NR²⁶-, wobei R²⁶ ausgewählt ist aus der Gruppe, bestehend aus H, C₁₋₆-Alkyl und Benzyl; und W ausgewählt ist aus der Gruppe, bestehend aus: 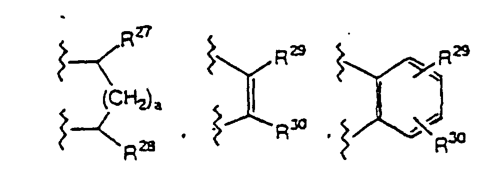 und einem - heterocyclischen C₆₋₁₀-Ringsystem, welches substituiert ist mit R²⁹ und R³⁰ und 1-4 Heteroatome enthält, die ausgewählt sind aus N, S und O; wobei a eine ganze Zahl von 0-2 ist; R²⁷ und R²⁸ unabhängig ausgewählt sind aus der Gruppe, bestehend aus H, C₁₋₆-Alkyl, Aryl, C₁₋₆-Alkylaryl, -COOR³¹, -CONR³¹R³², -CN und -CF₃; und R²⁹ und R³⁰ unabhängig ausgewählt sind aus der Gruppe, bestehend aus H, C₁₋₆-Alkyl, Aryl, C₁₋₆-Alkylaryl, C₁₋₄-Alkyloxy, Halogen, -NO₂, -NR³¹R³², -NR³¹COR³², -OR³¹, -OCOR³¹,-COOR³¹, -CONR³¹R³², -CN, -CF₃, -SO₂NR³¹R³² und C₁₋₆-Alkyl-OR³¹; wobei R³¹ und R³² unabhängig ausgewählt sind aus der Gruppe, bestehend aus H, C₁₋₆-Alkyl, C₁₋₃-Alkylaryl und Aryl;
und alle pharmazeutisch akzeptablen Salze und optischen Isomeren davon.

56. Pharmazeutische Zusammensetzung zum Vorbeugen oder Behandeln eines Zustands in einem Säugetier, der durch eine unerwünschte Thrombose charakterisiert ist, umfassend einen pharmazeutisch akzeptablen Träger und die Verbindung gemäß Anspruch 1.

57. Verwendung einer therapeutisch wirksamen Menge der Verbindung gemäß Anspruch 1 zur Herstellung eines Arzneimittels zum Vorbeugen oder Behandeln eines Zustands in einem Säugetier, der durch eine unerwünschte Thrombose charakterisiert ist.

58. Verwendung nach Anspruch 57, wobei der Zustand aus der Gruppe ausgewählt ist, bestehend aus: der Behandlung oder Vorbeugung von instabiler Angina, refraktärer Angina, Myocardinfarkt, vorübergehenden ischämischen Anfällen, Thromboseanfall, Embolieanfall, disseminierter intravaskulärer Gerinnung einschließlich der Behandlung eines septischen Shocks, tiefer Venenthrombose bei der Vorbeugung von Lungenembolie oder der Behandlung bei einem Wiederverschluß von Gefäßen oder einer Restenose von wiederperfundierten Koronararterien, tiefer Venenthrombose, Lungenembolie, Myocardinfarkt, Schlaganfall, thromboembolischen Komplikationen in der Chirurgie und bei einem peripheren Arterienverschluß, verstopfender Koronarthrombusbildung als Ergebnis einer thrombolytischen Therapie oder einer perkutenen Transluminalkoronarangioplastik, Thrombusbildung in der venösen Gefäßversorgung und disseminierter intravaskuläre Koagulopathie.

59. Verfahren zur Inhibierung der Koagulation von biologischen Proben, umfassend die Verabreichung der Verbindung gemäß Anspruch 1.

## Revendications

1. Composé ayant la formule : 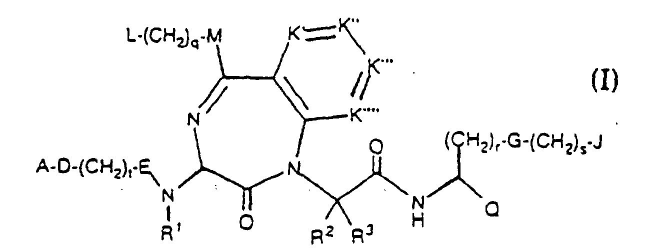 dans laquelle :
- R¹ et R² sont indépendamment choisis au sein du groupe comprenant les groupes H, alkyle C₁₋₆, cycloalkyle C₃₋₈, alkyl C₁₃ aryle, alkyl C₁₋₃-cycloalkyle C₃₋₈ et aryle ;
- R³ représente H, un groupe alkyle C₁₋₆ ou bien R² et R³ sont pris ensemble pour former un cycle carbocyclique ;
- q est un entier variant de 0 à 1 ;
- r est un entier variant de 0 à 4 ;
- s est un entier variant de 0 à 1 ;
- t est un entier variant de 0 à 4 ;
- A est choisi au sein du groupe comprenant R⁸, -NR⁸R⁹, 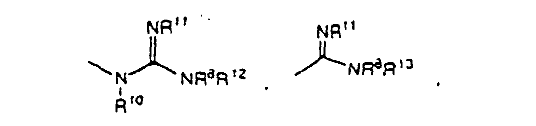 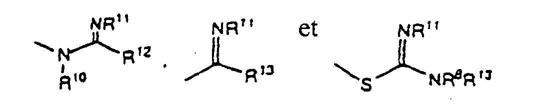 où R⁸, R⁹, R¹⁰ et R¹¹ sont indépendamment choisis au sein du groupe comprenant les groupes H, -OH, alkyle C₁₋₆, aryle et alkyl C₁₋₄ aryle ; R¹² est choisi au sein du groupe comprenant les groupes H, alkyle C₁₋₆, aryle et alkyl C₁₋₄ aryle, ou bien il peut être pris en association avec R¹⁰ ou R¹¹ pour former un cycle à 5 ou 6 atomes ; et R¹³ est choisi au sein du groupe comprenant les groupes H, alkyle C₁₋₆, aryle et alkyl C₁₋₄ aryle ou bien il peut être pris en association avec R¹¹ pour former un cycle à 5 ou 6 atomes ;
- D est choisi au sein du groupe comprenant une liaison directe, les groupes cycloalkyle C₃₋₈, alkényle C₁₋₆, alkényl C₁₋₆ aryle, aryle et un système de cycles hétérocycliques à cinq à dix atomes contenant 1 à 4 hétéroatomes choisis au sein du groupe comprenant N, O et S ;
- E est choisi au sein du groupe comprenant une liaison directe, les groupes -CO-, -SO₂-, -O-CO-, -NR¹⁴-SO₂- et -NR¹⁴-CO-, où R¹⁴ est choisi au sein du groupe comprenant les groupes H, -OH, alkyle C₁₋₆, aryle et alkyl C₁₋₄ aryle ;
- G est choisi au sein du groupe comprenant une liaison directe, les groupes cycloalkyle C₃₋₈, aryle et un système de cycles hétérocycliques à cinq à dix atomes contenant 1 à 4 hétéroatomes choisis au sein du groupe comprenant N, O et S ;
- J est choisi au sein du groupe comprenant les groupes R¹⁵, -NR¹⁵R¹⁶, 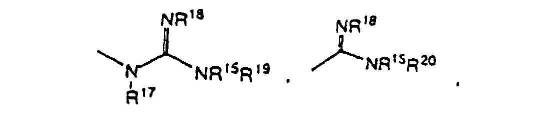 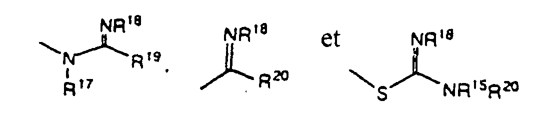 où R¹⁵, R¹⁶, R¹⁷ et R¹⁸ sont choisis indépendamment au sein du groupe comprenant les groupes H, -OH, alkyle C₁₋₆, aryle et alkyl C₁₋₄ aryle ; R¹⁹ est choisi au sein du groupe comprenant les groupes H, alkyle C₁₋₆, aryle et alkyl C₁₋₄ aryle, ou bien il peut être pris en association avec R¹⁷ ou R¹⁸ pour former un cycle à 5 ou 6 atomes ; et R²⁰ est choisi au sein du groupe comprenant les groupes H, alkyle C₁₋₆, aryle et alkyl C₁₋₄ aryle ou bien il peut être pris en association avec R¹⁸ pour former un cycle à 5 ou 6 atomes ; à la condition que lorsque J représente R¹⁵, G doive alors contenir au moins un atome N ;
- K', K", K"' et K"" sont indépendamment choisis au sein du groupe comprenant les groupes -CH-, -CR⁴-, -CR⁵- et -N- ; à la condition que pas plus d'un seul parmi K', K", K"' et K"" ne représente -CR⁴- et que pas plus d'un seul parmi K', K", K"' et K"" ne représente -C R⁵-;
- R⁴ et R⁵ sont choisis indépendamment au sein du groupe comprenant les groupes H, alkyle C₁₋₆, aryle, alkyl C₁₋₄ aryle, alkyloxy C₁₋₄, halogène, -NO₂, -NR⁶R⁷, -NR⁶COR⁷, -OR⁶, -OCOR⁶, -COOR⁶, -CONR⁶R⁷, -CN, -CF₃, -SO₂NR⁶R⁷ et alkyl C₁₋₆-OR⁶; où R⁶ et R⁷ sont choisis indépendamment au sein du groupe comprenant les groupes H, alkyle C₁₋₆, alkyl C₁₋₃ aryle et aryle ;
- L est choisi au sein du groupe comprenant les groupes R³³, -NR³³R³⁴, 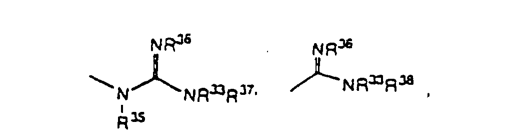 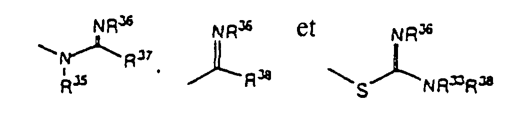 où R³³, R³⁴, R³⁵ et R³⁶ sont choisis indépendamment au sein du groupe comprenant les groupes H, -OH, alkyle C₁₋₆, aryle et alkyl C₁₋₄ aryle ; R³⁷ est choisi au sein du groupe comprenant les groupes H, alkyle C₁₋₆, aryle et alkyl C₁₋₄ aryle, ou bien il peut être pris en association avec R³⁵ ou R³⁶ pour former un cycle à 5 ou 6 atomes ; et R³⁸ est choisi au sein du groupe comprenant les groupes H, alkyle C₁₋₆, aryle et alkyl C₁₋₄ aryle, ou bien il peut être pris en association avec R³⁶ pour former un cycle à 5 ou 6 atomes ;
- M est choisi au sein du groupe comprenant une liaison directe, les groupes alkyle C₁₋₆, cycloalkyle C₃₋₈, alkényle C₁₋₆, alkényl C₁₋₆ aryle, et un système de cycles hétérocycliques à cinq à dix atomes contenant 1 à 4 hétéroatomes choisis au sein du groupe comprenant N, O et S ;
- Q est choisi au sein du groupe comprenant les groupes H, 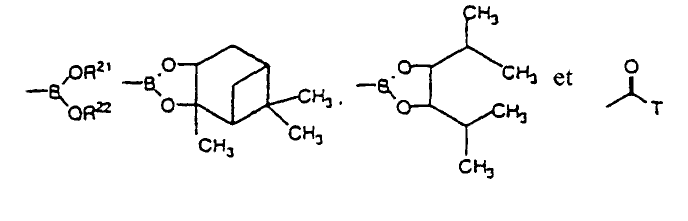 où R²¹ et R²² sont choisis indépendamment au sein du groupe comprenant les groupes H, alkyle C₁₋₃ et aryle ; et
- T est choisi au sein du groupe comprenant les groupes H, -COOR²³, -CONR²³R²⁴, -CF₃, -CF₂CF₃ et un groupe ayant la formule : 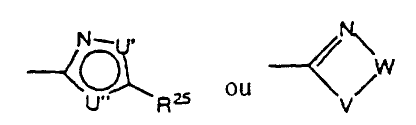 où R²³ et R²⁴ sont choisis indépendamment au sein du groupe comprenant les groupes H, alkyle C₁₋₆, aryle et alkyl C₁₋₄ aryle ; U' et U" sont choisis indépendamment au sein du groupe comprenant les groupes -O-, -S-, -N- et -NH-; à la condition qu'au moins l'un de U' ou de U" représente -N- ou -NH-; R²⁵ est choisi au sein du groupe comprenant les groupes H, alkyle C₁₋₆, akényle C₂₋₆, alkyl C₀₋₆ aryle, alkényl C₂₋₆ aryle, alkyl C₀₋₆ hétérocyclo, alkényl C₂₋₆ hétérocyclo, -CF₃ et -CF₂CF₃; V est choisi au sein du groupe comprenant les groupes -S-, -SO-, -SO₂-, -O- et -NR²⁶-, où R²⁶ est choisi au sein du groupe comprenant les groupes H, alkyle C₁₋₆ et benzyle ; et W est choisi au sein du groupe comprenant : 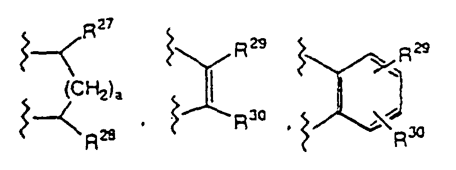 et un système de cycles hétérocycliques C₆₋₁₀ substitués par R²⁹ et R³⁰ et contenant 1 à 4 hétéroatomes choisis parmi N, S et O ; où a est un entier variant de 0 à 2 ; R²⁷ et R²⁸ sont choisis indépendamment au sein du groupe comprenant les groupes H, alkyle C₁₋₆, aryle, alkyl C₁₋₆ aryle, -COOR³¹, -CONR³¹R³², -CN et -CF₃ ; et R²⁹ et R³⁰ sont choisis indépendamment au sein du groupe comprenant les groupes H, alkyle C₁₋₆, aryle, alkyl C₁₋₆ aryle, alkyloxy C₁₋₄, halogène, -NO₂, -NR³¹R³², -NR³¹COR³², -OR³¹, -OCOR³¹, -COOR³¹, -CONR³¹R³², -CN, -CF₃, -SO₂NR³¹R³² et alkyl C₁₋₆-OR³¹ où R³¹ et R³² sont choisis indépendamment au sein du groupe comprenant les groupes H, alkyle C₁₋₆, alkyl C₁₋₃ aryle et aryle ;
et tous les sels et isomères optiques pharmaceutiquement acceptables de celui-ci.

2. Composé selon la revendication 1, dans, lequel R¹ représente H ou un groupe alkyle C₁₋₆.

3. Composé selon la revendication 2, dans lequel R' représente H ou un groupe méthyle.

4. Composé selon la revendication 3, dans lequel R¹ représente H.

5. Composé selon la revendication 1, dans lequel R² représente H ou un groupe alkyle C₁₋₆.

6. Composé selon la revendication 5, dans lequel R² représente H ou un groupe méthyle.

7. Composé selon la revendication 6, dans lequel R² représente H.

8. Composé selon la revendication 1, dans lequel R³ représente H.

9. Composé selon la revendication 1, dans lequel R⁴ représente un halogène.

10. Composé selon la revendication 1, dans lequel R⁵ représente un halogène.

11. Composé selon la revendication 1, dans lequel l'entier « r » est égal à 3.

12. Composé selon la revendication 1, dans lequel l'entier « s » est égal à 0.

13. Composé selon la revendication 1, dans lequel l'entier « t » est égal à 0 ou à 1.

14. Composé selon la revendication 1, dans lequel R⁸, R⁹, R¹⁰ et R¹¹ sont choisis indépendamment au sein du groupe comprenant les groupes H et alkyle C₁₋₆.

15. Composé selon la revendication 1, dans lequel R⁸, R⁹, R¹⁰ et R¹¹ sont choisis indépendamment au sein du groupe comprenant les groupes H et méthyle.

16. Composé selon la revendication 1, dans lequel R¹² représente H, un groupe alkyle C₁₋₆ ou bien est pris en association avec R¹⁰ ou R¹¹ pour former un cycle à 5 ou 6 atomes.

17. Composé selon la revendication 16, dans lequel R¹² représente H ou un groupe méthyle.

18. Composé selon la revendication 1, dans lequel R¹³ représente H, un groupe alkyle C₁₋₆ ou bien est pris en association avec R¹⁰ pour former un cycle à 5 ou 6 atomes.

19. Composé selon la revendication 18, dans lequel R¹³ représente H ou un groupe méthyle.

20. Composé selon la revendication 1, dans lequel D est choisi au sein du groupe comprenant une liaison directe, les groupes cycloalkyle C₃₋₈, aryle et un système de cycles hétérocycliques à cinq à dix atomes contenant 1 à 4 hétéroatomes choisis au sein du groupe comprenant N, O et S.

21. Composé selon la revendication 1, dans lequel E représente une liaison directe, les groupes -CO- ou -SO₂-.

22. Composé selon la revendication 1, dans lequel G représente une liaison directe.

23. Composé selon la revendication 1, dans lequel R¹⁵, R¹⁶, R¹⁷, R¹⁸, R¹⁹ et R²⁰ sont choisis indépendamment au sein du groupe comprenant H et le groupe alkyle C₁₋₆.

24. Composé selon la revendication 23, dans lequel R¹⁵, R¹⁶, R¹⁷, R¹⁸, R¹⁹ et R²⁰ sont choisis indépendamment au sein du groupe comprenant H et le groupe méthyle.

25. Composé selon la revendication 1, dans lequel au moins trois parmi K', K", K"' et K"" représentent le groupe -CH-.

26. Composé selon la revendication 25, dans lequel K', K", K"' et K"" représentent le groupe -CH-.

27. Composé selon la revendication 1, dans lequel R³³, R³⁴, R³⁵ et R³⁶ sont choisis indépendamment au sein du groupe comprenant H et le groupe alkyle C₁₋₆.

28. Composé selon la revendication 27, dans lequel R³³, R³⁴, R³⁵ et R³⁶ sont choisis indépendamment au sein du groupe comprenant H et le groupe méthyle.

29. Composé selon la revendication 1, dans lequel R³⁷ représente H, un groupe alkyle C₁₋₆, ou bien est pris en association avec R³⁵ ou R³⁶ pour former un cycle à 5 ou 6 atomes.

30. Composé selon la revendication 29, dans lequel R³⁷ représente H ou un groupe méthyle.

31. Composé selon la revendication 1, dans lequel R³⁸ représente H, un groupe alkyle C₁₋₆, ou bien est pris en association avec R³⁶ pour former un cycle à 5 ou 6 atomes.

32. Composé selon la revendication 31, dans lequel R³⁸ représente H ou un groupe méthyle.

33. Composé selon la revendication 1, dans lequel M représente une liaison directe, un groupe alkyle C₁₋₆, cycloalkyle C₃₋₈, aryle ou un système de cycles hétérocycliques à cinq à dix atomes.

34. Composé selon la revendication 33, dans lequel M représente un groupe alkyle C₁₋₄, aryle ou un système de cycles hétérocycliques à cinq à dix atomes.

35. Composé selon la revendication 1, dans lequel l'entier q est égal à 0.

36. Composé selon la revendication 1, dans lequel Q représente :

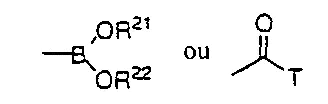

37. Composé selon la revendication 36, dans lequel R²¹ représente H.

38. Composé selon la revendication 37, dans lequel R²² représente H.

39. Composé selon la revendication 36, dans lequel T représente H, un groupe -COOR²³, -CONR²³R²⁴ ou un groupe ayant la formule :

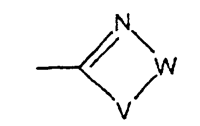

40. Composé selon la revendication 39, dans lequel R²³ représente H.

41. Composé selon la revendication 39, dans lequel R²⁴ représente un groupe alkyl C₁₋₄ aryle.

42. Composé -selon la revendication 39, dans lequel V représente un groupe -S-, -O- ou -NR²⁶.

43. Composé selon la revendication 42, dans lequel R²⁶ représente H ou un groupe méthyle.

44. Composé selon la revendication 43, dans lequel R²⁶ représente H.

45. Composé selon la revendication 39, dans lequel W est choisi au sein du groupe comprenant

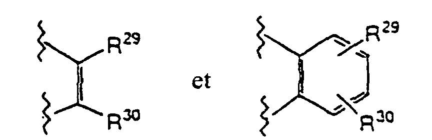

46. Composé selon la revendication 45, dans lequel W représente le groupe :

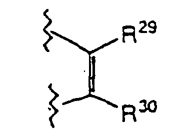

47. Composé selon la revendication 45, dans lequel R²⁹ est choisi au sein du groupe comprenant H, les groupes -O-COOR³¹, -COOR³¹, -CON R³¹R³² ou -CF₃.

48. Composé selon la revendication 47, dans lequel R²⁹ représente H.

49. Composé selon la revendication 45, dans lequel R³⁰ est choisi au sein du groupe comprenant H, les groupes -O-R³¹, -COOR³¹, -CONR³¹R³², ou -CF₃.

50. Composé selon la revendication 49, dans lequel R³⁰ représente H.

51. Composé selon la revendication 39, dans lequel W représente le groupe: 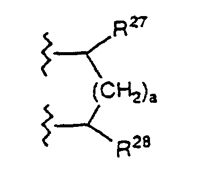 et R²⁷ représente H.

52. Composé selon la revendication 51, dans lequel R²⁸ représente H.

53. Composé selon la revendication 36, dans lequel T représente le groupe: 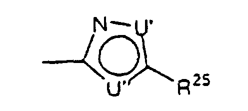 U' représente O, U" représente N et R²⁵ représente un groupe -CF₃ ou -CF₂CF₃.

54. Composé ayant la formule : 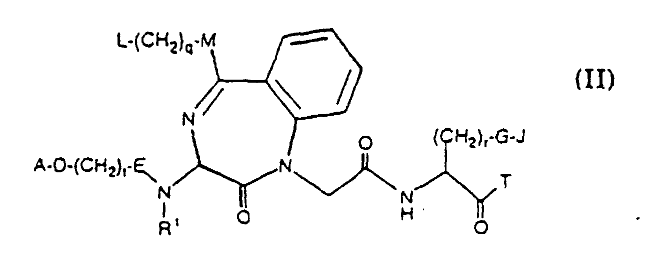 dans laquelle
- R¹ est choisi au sein du groupe comprenant les groupes H, alkyle C₁₋₆, cycloalkyle C₃₋₈, alkyl C₁₋₃ aryle, alkyl C₁₋₃-cycloalkyle C₃₋₈ et aryle ;
- q est un entier variant de 0 à 1 ;
- r est un entier variant de 0 à 4 ;
- t est un entier variant de 0 à 4 ;
- A est choisi au sein du groupe comprenant R⁸, -NR⁸R⁹, 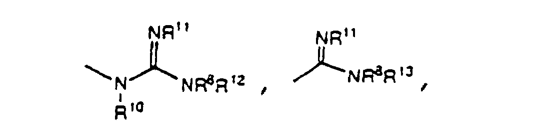 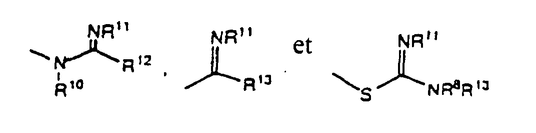 où R⁸, R⁹, R¹⁰ et R¹¹ sont indépendamment choisis au sein du groupe comprenant les groupes H, -OH, alkyle C₁₋₆, aryle et alkyl C₁₋₄ aryle; R¹² est choisi au sein du groupe comprenant les groupes H, alkyle C₁₋₆, aryle et alkyl C₁₋₄ aryle, ou bien il peut être pris en association avec R¹⁰ ou R¹¹ pour former un cycle à 5 ou 6 atomes ; et R¹³ est choisi au sein du groupe comprenant les groupes H, alkyle C₁₋₆, aryle et alkyl C₁₋₄ aryle ou bien il peut être pris en association avec R¹¹ pour former un cycle à 5 ou 6 atomes ;
- D est choisi au sein du groupe comprenant une liaison directe, les groupes cycloalkyle C₃₋₈, alkényle C₁₋₆, alkényl C₁₋₆ aryle, aryle et un système de cycles hétérocycliques à cinq à dix atomes contenant 1 à 4 hétéroatomes choisis au sein du groupe comprenant N, O et S ;
- E est choisi au sein du groupe comprenant une liaison directe, les groupes -CO-, -SO₂-, -O-CO-, -NR¹⁴-SO₂- et -NR¹⁴-CO-, où R¹⁴ est choisi au sein du groupe comprenant les groupes H, -OH, alkyle C₁₋₆, aryle et alkyl C₁₋₄ aryle ;
- G est choisi au sein du groupe comprenant une liaison directe, les groupes cycloalkyle C₃₋₈, aryle et un système de cycles hétérocycliques à cinq à dix atomes contenant 1 à 4 hétéroatomes choisis au sein du groupe comprenant N, O et S ;
- J est choisi au sein du groupe comprenant les groupes R¹⁵, -NR¹⁵R¹⁶, 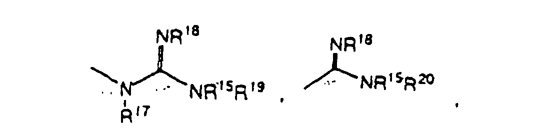 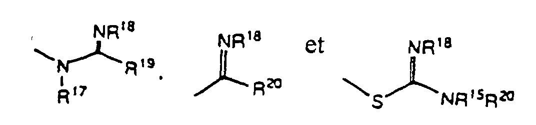 où R¹⁵, R¹⁶, R¹⁷ et R¹⁸ sont choisis indépendamment au sein du groupe comprenant les groupes H, -OH, alkyle C₁₋₆, aryle et alkyl C₁₋₄ aryle ; R¹⁹ est choisi au sein du groupe comprenant les groupes H, alkyle C₁₋₆, aryle et alkyl C₁₋₄ aryle, ou bien il peut être pris en association avec R¹⁷ ou R¹⁸ pour former un cycle à 5 ou 6 atomes ; et R²⁰ est choisi au sein du groupe comprenant les groupes H, alkyle C₁₋₆, aryle et alkyl C₁₋₄ aryle ou bien il peut être pris en association avec R¹⁸ pour former un cycle à 5 ou 6 atomes ; à la condition que lorsque J représente R¹⁵, G doive alors contenir au moins un atome N ;
- L est choisi au sein du groupe comprenant les groupes R³³, -NR³³R³⁴, 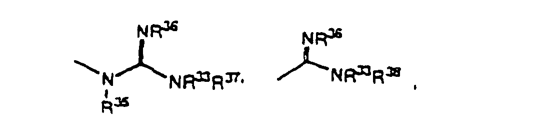 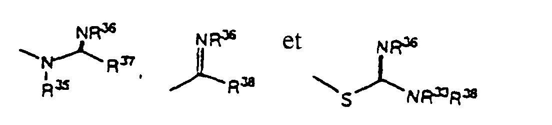 où R³³, R³⁴, R³⁵ et R³⁶ sont choisis indépendamment au sein du groupe comprenant les groupes H, -OH, alkyle C₁₋₆, aryle et alkyl C₁₋₄ aryle ; R³⁷ est choisi au sein du groupe comprenant les groupes H, alkyle C₁₋₆, aryle et alkyl C₁₋₄ aryle, ou bien il peut être pris en association avec R³⁵ ou R³⁶ pour former un cycle à 5 ou 6 atomes ; et R³⁸ est choisi au sein du groupe comprenant les groupes H, alkyle C₁₋₆, aryle et alkyl C₁₋₄ aryle, ou bien il peut être pris en association avec R³⁶ pour former un cycle à 5 ou 6 atomes ;
- M est choisi au sein du groupe comprenant une liaison directe, les groupes alkyle C₁₋₆, cycloalkyle C₃₋₈, alkényle C₁₋₆, alkényl C₁₋₆ aryle, et un système de cycles hétérocycliques à cinq à dix atomes contenant 1 à 4 hétéroatomes choisis. au sein du groupe comprenant N, O et S ;
- T est choisi au sein du groupe comprenant les groupes H, -COOR²³, -CONR²³R²⁴, -CF₃, -CF₂CF₃ et un groupe ayant la formule : 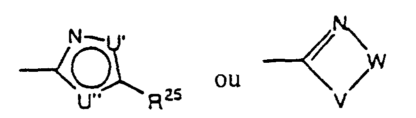 où R²³ et R²⁴ sont choisis indépendamment au sein du groupe comprenant les groupes H, alkyle C₁₋₆, aryle et alkyl C₁₋₄ aryle ; U' et U" sont choisis indépendamment au sein du groupe comprenant les groupes -O-, -S-, -N- et -NH-; à la condition qu'au moins l'un de U' ou de U" représente -N- ou -NH-; R²⁵ est choisi au sein du groupe comprenant les groupes H, alkyle C₁₋₆, akényle C₂₋₆, alkyl C₀₋₆ aryle, alkényl C₂₋₆ aryle, alkyl C₀₋₆ hétérocyclo, alkényl C₂₋₆ hétérocyclo, -CF₃ et -CF₂CF₃ ; V est choisi au sein du groupe comprenant les groupes -S-, -SO-, -SO₂-, -O- et -NR²⁶-, où R²⁶ est choisi au sein du groupe comprenant les groupes H, alkyle C₁₋₆ et benzyle ; et W est choisi au sein du groupe comprenant : 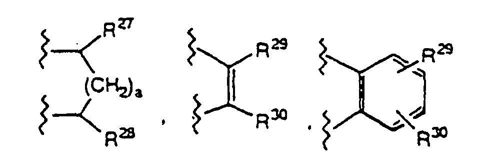 et un système de cycles hétérocycliques C₆₋₁₀ substitués par R²⁹ et R³⁰ et contenant 1 à 4 hétéroatomes choisis parmi N, S et O ; où a est un entier variant de 0 à 2 ; R²⁷ et R²⁸ sont choisis indépendamment au sein du groupe comprenant les groupes H, alkyle C₁₋₆, aryle, alkyl C₁₋₆ aryle, -COOR³¹, -CONR³¹R³², -CN et -CF₃ ; et R²⁹ et R³⁰ sont choisis indépendamment au sein du groupe comprenant les groupes H, alkyle C₁₋₆, aryle, alkyl C₁₋₆ aryle, alkyloxy C₁₋₄, halogène, -NO₂, -NR³¹R³², -NR³¹COR³², -OR³¹, -OCOR³¹, -COOR³¹, -CONR³¹R³², -CN, -CF₃, -SO₂NR³¹R³² et alkyl C₁₋₆-OR³¹ où R³¹ et R³² sont choisis indépendamment au sein du groupe comprenant les groupes H, alkyle C₁₋₆, alkyl C₁₋₃ aryle et aryle ;
et tous les sels et isomères optiques pharmaceutiquement acceptables de - celui-ci.

55. Composé ayant la formule : 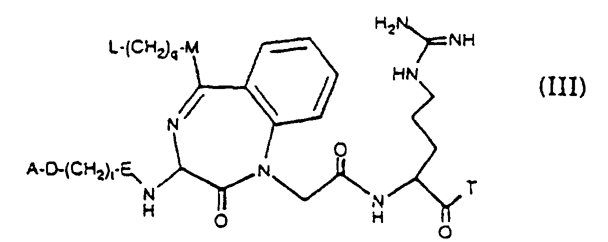 dans laquelle :
- q est un entier variant de 0 à 1 ;
- t est un entier variant de 0 à 4 ;
- A est choisi au sein du groupe comprenant R⁸, -NR⁸R⁹, 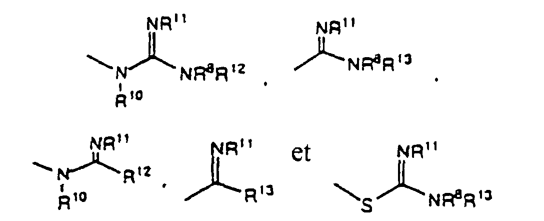 où R⁸, R⁹, R¹⁰ et R¹¹ sont indépendamment choisis au sein du groupe comprenant les groupes H, -OH, alkyle C₁₋₆, aryle et alkyl C₁₋₄ aryle ; R¹² est choisi au sein du groupe comprenant les groupes H, alkyle C₁₋₆, aryle et alkyl C₁₋₄ aryle, ou bien il peut être pris en association avec R¹⁰ ou R¹¹ pour former un cycle à 5 ou 6 atomes ; et R¹³ est choisi au sein du groupe comprenant les groupes H, alkyle C₁₋₆, aryle et alkyl C₁₋₄ aryle ou bien il peut être pris en association avec R¹¹ pour former un cycle à 5 ou 6 atomes ;
- D est choisi au sein du groupe comprenant une liaison directe, les groupes cycloalkyle C₃₋₈, alkényle C₁₋₆, alkényl C₁₋₆ aryle, aryle et un système de cycles hétérocycliques à cinq à dix atomes contenant 1 à 4 hétéroatomes choisis au sein du groupe comprenant N, O et S ;
- E est choisi au sein du groupe comprenant une liaison directe, les groupes -CO-, -SO₂-, -O-CO-, -NR¹⁴-SO₂- et -NR¹⁴-CO-, où R¹⁴ est choisi au sein du groupe comprenant les groupes H, -OH, alkyle C₁₋₆, aryle et alkyl C₁₋₄ aryle ;
- L est choisi au sein du groupe comprenant les groupes R³³, -NR³³R³⁴, 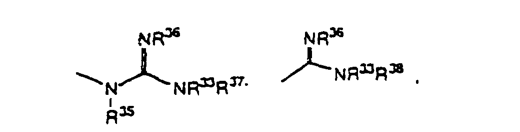 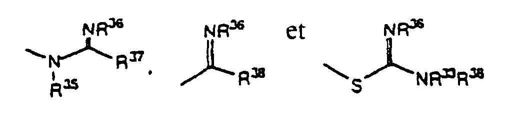 où R³³, R³⁴, R³⁵ et R³⁶ sont choisis indépendamment au sein du groupe comprenant les groupes H, -OH, alkyle C₁₋₆, aryle et alkyl C₁₋₄ aryle ; R³⁷ est choisi au sein du groupe comprenant les groupes H, alkyle C₁₋₆, aryle et alkyl C₁₋₄ aryle, ou bien il peut être pris en association avec R³⁵ ou R³⁶ pour former un cycle à 5 ou 6 atomes ; et R³⁸ est choisi au sein du groupe comprenant les groupes H, alkyle C₁₋₆, aryle et alkyl C₁₋₄ aryle, ou bien il peut être pris en association avec R³⁶ pour former un cycle à 5 ou 6 atomes ;
- M est choisi au sein du groupe comprenant une liaison directe, les groupes alkyle C₁₋₆, cycloalkyle C₃₋₈, alkényle C₁₋₆, alkényl C₁₋₆ aryle, et un système de cycles hétérocycliques à cinq à dix atomes contenant 1 à 4 hétéroatomes choisis au sein du groupe comprenant N, O et S ;
- T est choisi au sein du groupe comprenant les groupes H, -COOR²³, -CONR²³R²⁴, -CF₃, -CF₂CF₃ et un groupe ayant la formule : 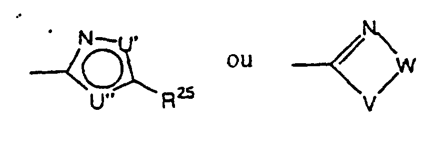 où R²³ et R²⁴ sont choisis indépendamment au sein du groupe comprenant les groupes H, alkyle C₁₋₆, aryle et alkyl C₁₋₄ aryle ; U' et U" sont choisis indépendamment au sein du groupe comprenant les groupes -O-, -S-, -N- et -NH- ; à la condition qu'au moins l'un de U' ou de U" représente -N- ou -NH-; R²⁵ est choisi au sein du groupe comprenant les groupes H, alkyle C₁₋₆, akényle C₂₋₆, alkyl C₀₋₆ aryle, alkényl C₂₋₆ aryle, alkyl C₀₋₆ hétérocyclo, alkényl C₂₋₆ hétérocyclo, -CF₃ et -CF₂CF₃; V est choisi au sein du groupe comprenant les groupes -S-, -SO-, -SO₂-, -O- et -NR²⁶-, où R²⁶ est choisi au sein du groupe comprenant les groupes H, alkyle C₁₋₆ et benzyle ; et W est choisi au sein du groupe comprenant : 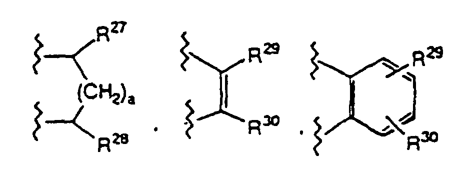 et un système de cycles hétérocycliques C₆₋₁₀ substitués par R²⁹ et R³⁰ et contenant 1 à 4 hétéroatomes choisis parmi N, S et O ; où a est un entier variant de 0 à 2 ; R²⁷ et R²⁸ sont choisis indépendamment au sein du groupe comprenant les groupes H, alkyle C₁₋₆, aryle, alkyl C₁₋₆ aryle, -COOR³¹, -CONR³¹R³², -CN et -CF₃ ; et R²⁹ et R³⁰ sont choisis indépendamment au sein du groupe comprenant les groupes H, alkyle C₁₋₆, aryle, alkyl C₁₋₆ aryle, alkyloxy C₁₋₄, halogène, -NO₂, -NR³¹R³², -NR³¹COR³², -OR³¹, -OCOR³¹, -COOR³¹, -CONR³¹R³², -CN, -CF₃, -SO₂NR³¹R³² et alkyl C₁₋₆-OR³¹ où R³¹ et R³² sont choisis indépendamment au sein du groupe comprenant les groupes H, alkyle C₁₋₆, alkyl C₁₋₃ aryle et aryle ;
et tous les sels et isomères optiques pharmaceutiquement acceptables de celui-ci.

56. Composition pharmaceutique destinée à la prévention ou au traitement chez un mammifère, d'un état **caractérisé par** une thrombose indésirable, comprenant un véhicule pharmaceutiquement acceptable et le composé selon la revendication 1.

57. Utilisation d'une quantité thérapeutiquement efficace du composé selon la revendication 1, pour la fabrication d'un médicament destiné à la prévention ou au traitement chez un mammifère, d'un état **caractérisé par** une thrombose indésirable.

58. Utilisation selon la revendication 57, dans laquelle l'état est choisi au sein du groupe comprenant: le traitement ou la prévention de l'angine instable, de l'angine réfractaire, de l'infarctus du myocarde, des attaques ischiémiques passagères, de l'attaque thrombotique, de l'attaque embolique, de la coagulation intravasculaire disséminée comprenant le traitement du choc septique, de la thrombose veineuse profonde dans la prévention de l'embolie pulmonaire ou le traitement de la ré-occlusion ou de la re-sténose des artères coronaires retransfusées, de la thrombose veineuse profonde, de l'embolie pulmonaire, de l'infarctus du myocarde, de l'attaque, des complications thrombo-emboliques en chirurgie et de l'occlusion artérielle périphérique, de la formation de thrombus coronaire occlusif résultant soit de la thérapie thrombolytique, soit de l'angioplastie coronaire transluminale percutanée, de la formation de thrombus dans le système vasculaire veineux et de la coagulopathie intravasculaire disséminée.

59. Technique d'inhibition de la coagulation d'échantillons biologiques comprenant l'administration du composé selon la revendication 1.
